# EUROPEAN PATENT APPLICATION

(11) **EP 1 394 715 A1**
(43) Date of publication of application: **03.03.2004**
(21) Application number: 02017411.6
(22) Date of filing: 02.08.2002
(51) Int. Cl.: G06F 19/00

(54) **An expert system for clinicial outcome prediction**

(71) Applicant: Europroteome AG, 16761 Hennigsdorf (DE)
(72) Inventor: Ragg, Thomas, Dr., 76356 Weingarten (DE)
(74) Representative: Hengelhaupt, Jürgen D., Dipl.-Ing.

(57) **Abstract**

The present invention generally relates to an expert system for clinical outcome prediction. In particular, the invention relates to a generic data-mining system for predicting the development of a disease and/or for identifying high-risk patients.

A method for predicting the development of a disease and/or identifying high-risk patients, and a system for performing the method, is provided, including the steps of providing molecular genetic data and/or clinical data, pre-processing the data, selecting a predetermined number of variables out of the provided data according to their combined/mutual information content, automatically generating prediction data by means of machine learning.

## Description

This invention relates to an expert system for clinical outcome prediction, in particular to a generic data-mining system for predicting the development of a disease and/or for identifying high-risk patients.

Colorectal cancer is the fourth commonest form of cancer worldwide. In Europe there was a general improvement in survival over the period 1978-1989 - from 40% to 48% for colon cancer and 38% to 46% for rectal cancer ¹ - possibly explained by better surgical outcome, in particular in rectal cancer, as well as by the introduction of routine adjuvant chemotherapy after curative surgery ². Chemotherapy was also effective in a palliative setting for prolonging time to disease progression and survival in patients with metastatic colorectal cancer ³.

For indicating such adjuvant therapy after curative surgery, current recommendations are based on the TNM (Tumor Node Metastasis) system, defining the anatomical extent of disease at the timepoint of diagnosis, and the R classification (defining the presence or absence of Residual disease after surgical resection), as defined by the American Joint Committee on Cancer and the Union Internationale Contre le Cancer (AJCC/UICC) ⁴. The majority of patients is diagnosed in the AJCC/UICC stage III with about 40% of them developing distant metastases. Thus, chemotherapy is recommended for stage III patients ⁵.

However, in AJCC/UICC stages I and II, a number of patients also develop metachronous metastases during the course of disease ⁶. In recent years, major research efforts have been aimed at the discovery of molecular features predictive of such distant metastases, to allow improved indication for adjuvant therapy. However, in our hands as in other, the observed differences in the survival rate for single genotypic or phenotypic landmarks ⁷⁻¹⁰, or a combination of markers ¹¹ were rather discouraging. A reason might be that multiple genetic changes exist within tumor cells and that, besides these tumor-related-factors, patient-and treatment-related parameters such the effect of age, sex, tumor site or the quality of surgery had not been considered enough in these studies.

Recently non-linear statistical methods were applied to predict the likehood of survival in colorectal cancer patients ¹²⁻¹⁴. These neural network based approaches delivered predictions superior to conventional logistic regression approaches, and more accurate than subjective clinical appraisal. However, they did not discover significant variables for determining high-risk patients for metastases within the staging groups. Furthermore, patients presenting with distant metastases, in which palliative chemotherapy is indicated anyway, were included into the prediction models ^{13,14}. Thus, further criteria needed to be established serving as a basis for selection of patients with a high risk of dying or of developing distant metastases after curative surgery for colorectal cancer.

In the W0 02/06829, a process for determining a biological state through the discovery and analysis of hidden or non-obvious, discriminatory biological data patterns is described.

It is an object underlying the present invention to provide a method, a computer program and a computer system for better predicting the development of a disease and/or identifying high-risk patients.

These objects are achieved with the subject-matter as recited in the claims and in the description.

To overcome the limitations of conventional staging and statistical approaches for clinical outcome prediction, a generic data-mining framework comprising a variety of machine learning techniques is provided. The present invention relates to a method and system for predicting the development of a disease, in particular tumors, and/or identifying high-risk patients with the following features: providing molecular genetic data and/or clinical data, pre-processing the data, selecting a predetermined number of variables out of the provided data according to their combined/mutual information content, and automatically generating prediction data by means of machine learning. The method of the present invention allows a dramatic improvement in patient-specific prediction of distant metastases and survival, as compared to conventional tumor staging.

The subject-matter according to the present invention and preferred embodiments thereof are further recited in the claims.

The invention and preferred embodiments will now be exemplified with reference to the figures:

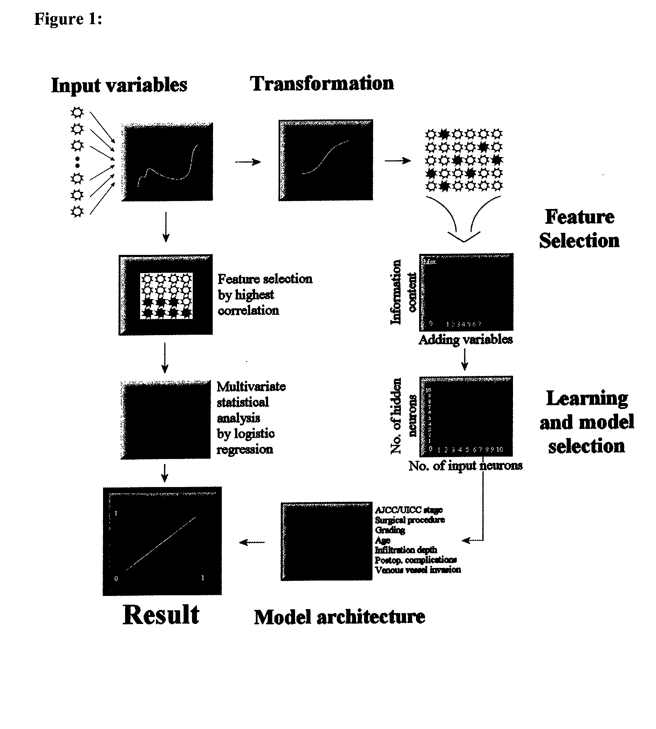

**Figure 1.** Schematized process flow of the analysis strategy applied (right part of the diagram), compared with a classical statistical workflow (left part). To prevent artificial non-linearity, symbolic values are converted in the ***Transformation*** step. The variable with the highest information content is selected first and subsequently one of the remaining variables is added iteratively *(****Feature selection****).* In the ***Learning and model selection*** -step several models are trained with different input vectors and number of hidden neurons, as computed by the Bayesian evidence approach. The best ***Model architecture*** for clinical outcome prediction (bottom right) is selected accordingly. The last step is ***Risk assignment,***

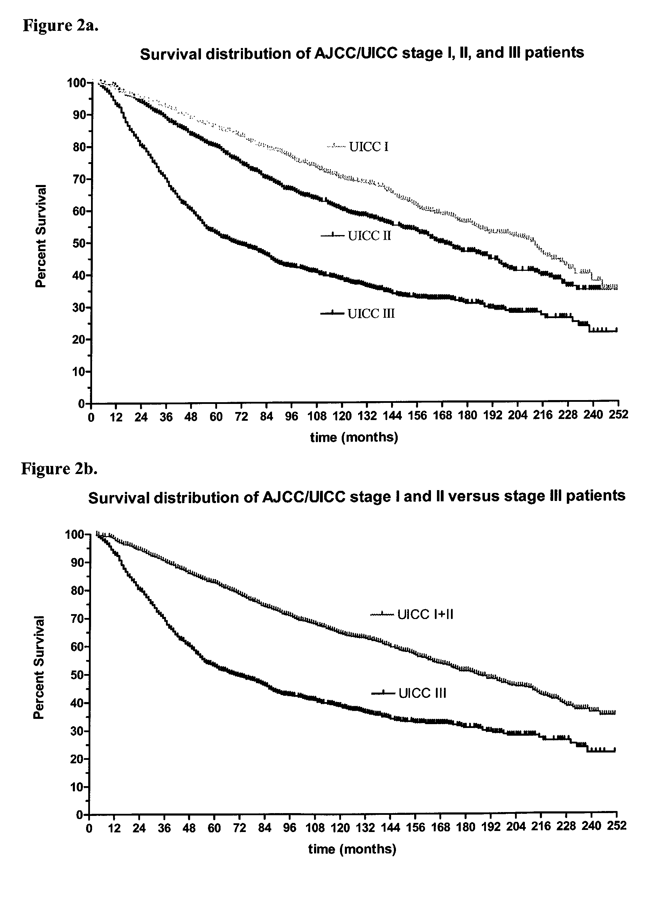

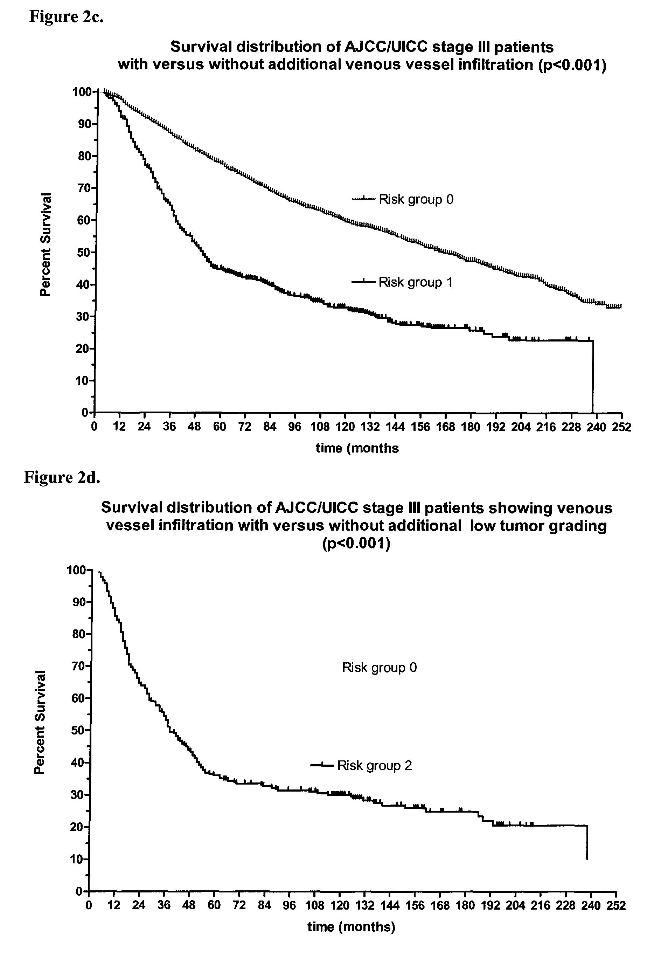

**Figure 2.** Stepwise definition of an individual risk profile (Kaplan-Meier survival plots for different patient groups) (a) observed survival for all AJCC/UICC stage III patients (b) Focus on a subgroup of patients (risk group 1) who additionally presented infiltration of venous vessels. Differences in survival of 33% after 5 years are observed (c) Focus on risk group 2, comprising patients who have confirmed tumor infiltration into venous vessels, and whose tumors presented low differentiation of tumor cells in the histopathological examination. Differences in crude survival of 38% after 5 years can now be observed within the same UICC stage. The difference observed between both populations with risk factors 1 and 2 vs. risk group 0 is highly significant (p<0.001) according to the Mantel-Haenszel test.

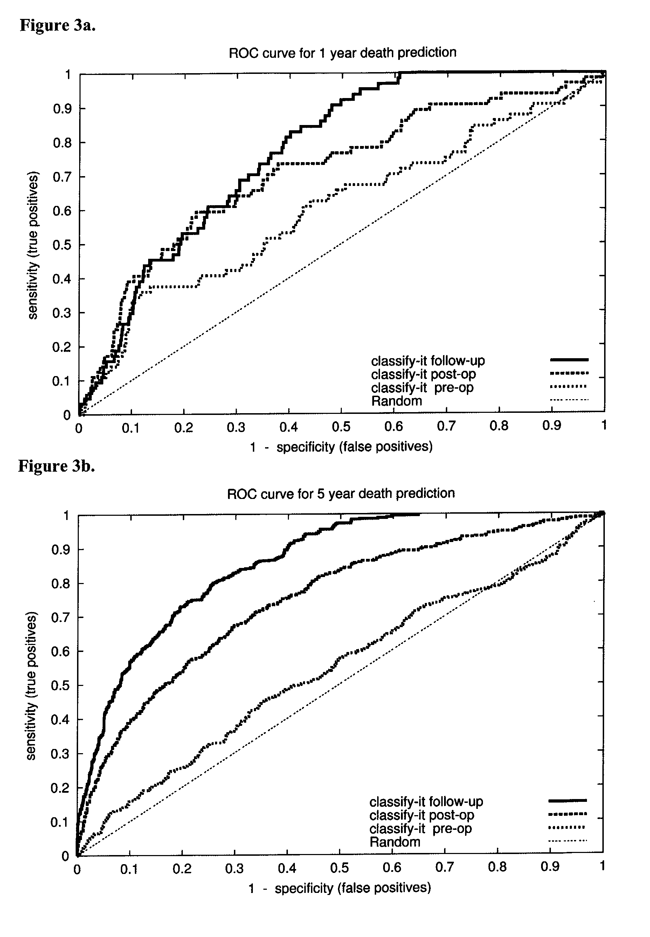

**Figure 3.** To improve prediction in stages I and II, feature selection and training of the classifier was now performed only within the corresponding stage. Kaplan-Meier survival plots for each stage are presented. (a) Stage I : Patients from the test data set were assigned to the high risk group (36 patients) resp. low risk group (449 patients) according to the prediction of our classifier (Classify-it). (b,c) For stage II (respectively stage III) Classify-it assigned 83 patients (360 patients) to the high-risk group, resp. 350 patients to the low-risk group. The difference between the two subgroups in all three stages is highly significant (p<0.001) according to a log-rank test. Thus, the system allowed the identification of high-risk patients stages I and II (and of low-risk patients in stage III).

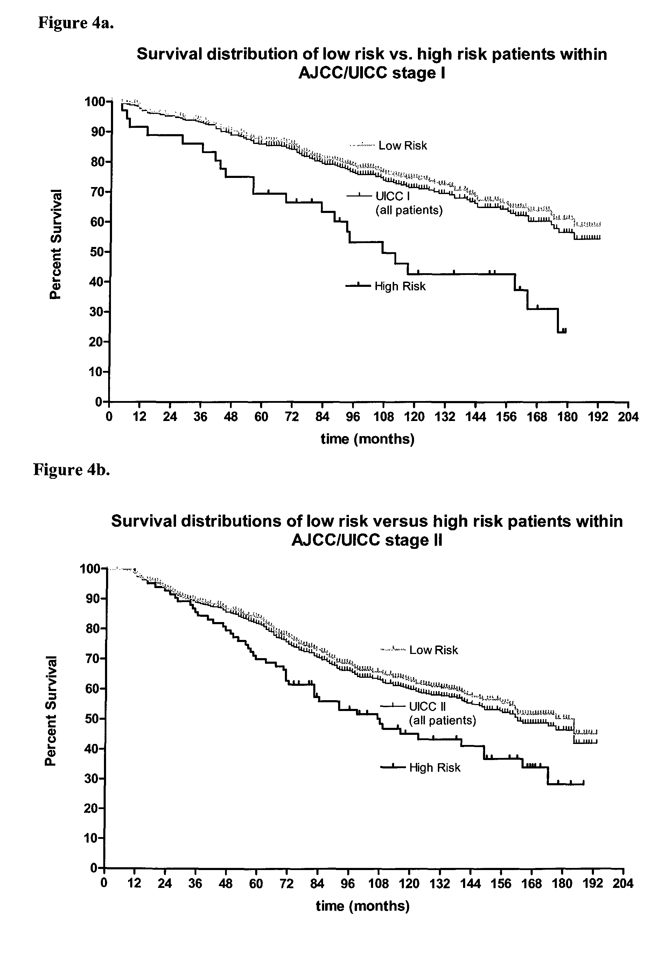

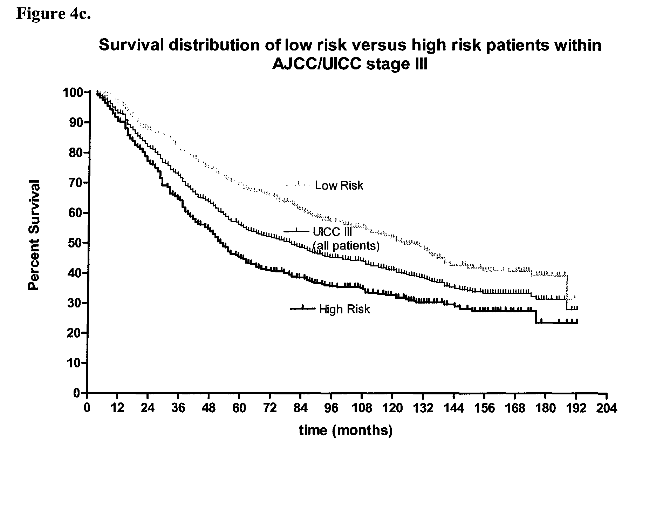

**Figure 4**. ROC curves are shown for 5-year death prediction predictions based on pre-operative (dotted lines) and post-operative (continuous lines) variables. ROC-value with pre-operative variables : 0.54, with postoperative variables 0.75. A ROC graph illustrates the prediction of true positive cases (sensitivity) versus the predicted false positives (1 - sensitivity).

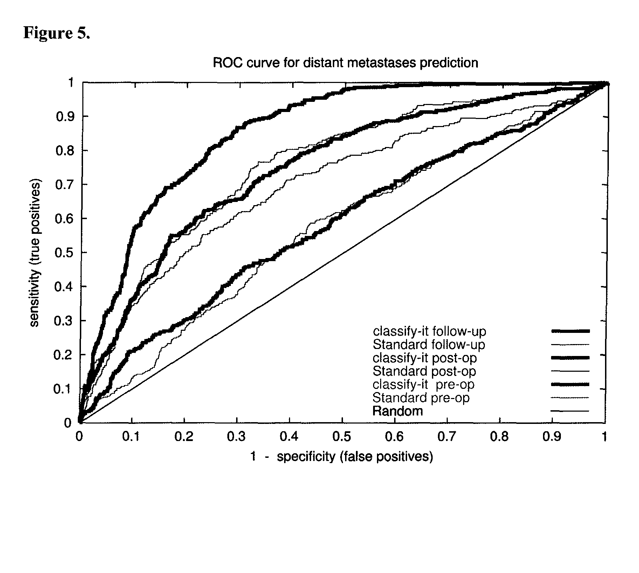

**Figure 5.** Benchmarking classify-it (thick lines) with standard classification by multivariate logistic regression (thin lines). ROC curves are shown for metastases predictions based on pre-operative (dotted lines) and post-operative (continuous lines). Differences are highly significant for post-operative variables (p<0.001), and still significant for pre-operative variables (p<0.03). ROC-value with pre-operative variables : Classify-it 0.58, standard (AJCC/UICC staging) 0.57; ROC-value with postoperative variables : Classify-it 0.76, standard 0.70

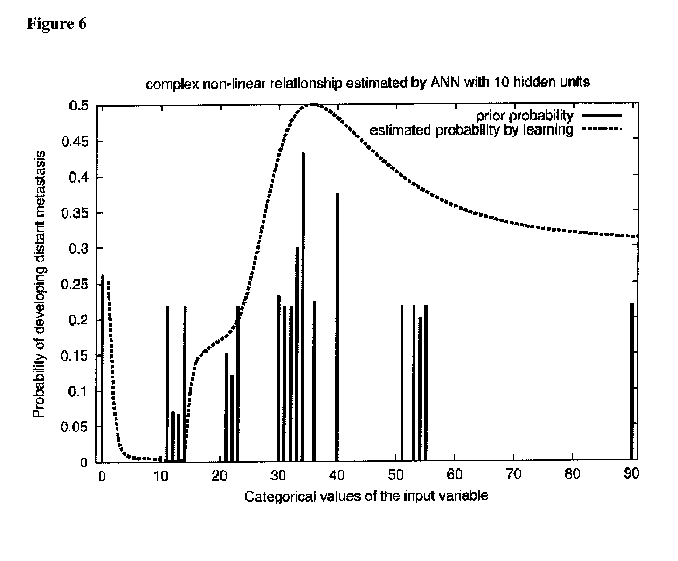

**Figure 6:** The complex non-linear relationship between the input parameters and the probability of developing distant metastasis is displayed both for the prior probability (filled line) as well as for the probability as estimated by an ANN with 10 hidden units (dashed line).

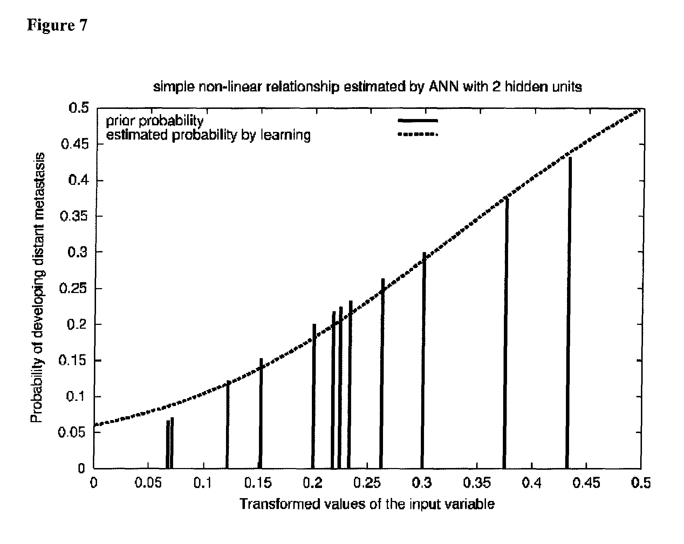

**Figure 7:** After transformation of the input variables the complex non-linear relationship (see supp. Fig. 1) is reduced to a more simple relationship. The relationship between the transformed input parameters and the probability of developing distant metastasis is displayed both for the prior probability (filled line) as well as for the probability as estimated by an ANN with 2 hidden units (dashed line).

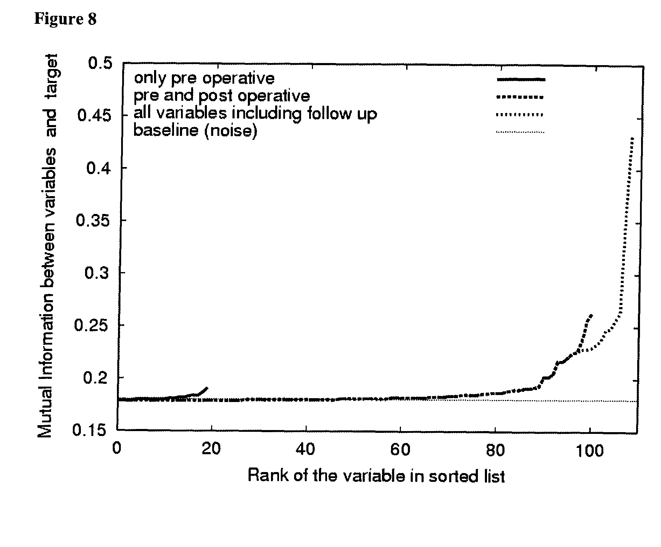

**Figure 8:** The mutual information between the ranked input variables and the target variable (probability of developing distant metastasis) is displayed for pre-operative, pre- and post-operative and all variables, respectively. The baseline (fine dotted line) is displayed for comparison.

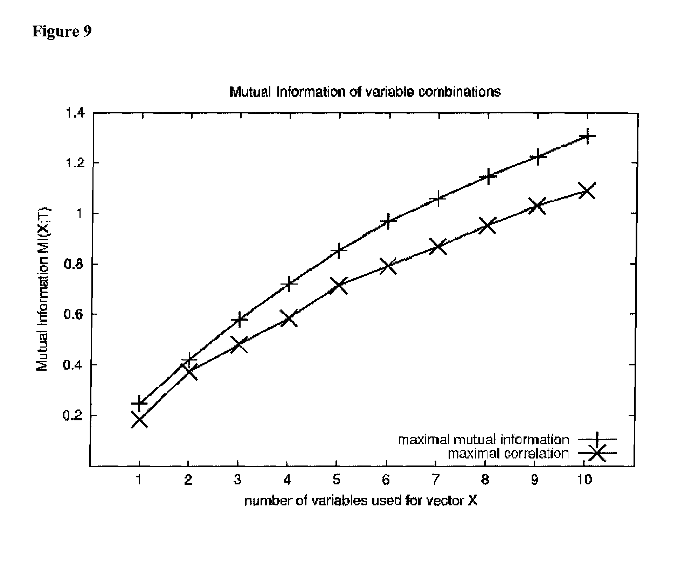

**Figure 9:** The mutual information for combinations of variables is displayed in dependence of number of combined variables. The mutual information (red) is compared to the values obtained by maximal correlation (green).

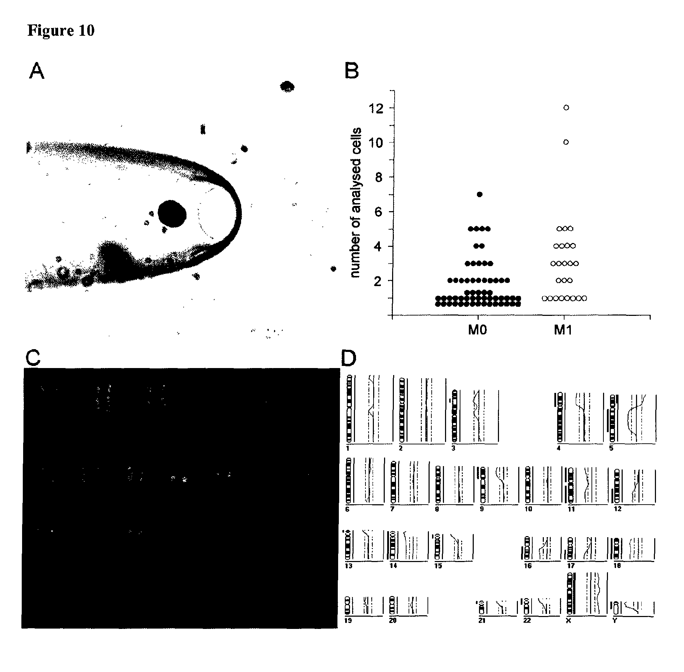

**Figure 10 Single cell analysis**
   **a**, Single cytokeratin-positive tumor cell is isolated by micromanipulation with a glass capillary. **b,** Number of analysed cells per patient isolated from the two clinical stages **c**, Sorted human metaphase chromosomes hybridized with labeled PCR product from a single tumor cell (green) and reference DNA (red). **d**, CGH-profile resulting from the hybridization shown in b. Chromosomal gains are indicated by a green bar on the right side and losses by red bar on the left side of the ideogram.
**Figure 11 Chromosomal aberrations of disseminated tumor cells**
   CGH aberrations for each chromosome of a, 40 disseminated tumor cells from 30 patients with minimal residual disease and b, of 47 single cells from 23 patients with clinical metastasis. Green and red bars indicate amplification and loss, respectively.
**Figure 12 Classification of chromosmal aberrations**
   a, Mean number of chromosomal gains and losses per cell that either affect the whole chromosome, a chromosome arm, a telomeric or an internal fragment for M0 and M1 cells. b, Example for each type of chromosomal aberration, showing the ideogram and the respective hybridization picture.
**Figure 13 ROC curve for M1 membership prediction**
   The percentage of truly positive classified M1-cells (y-axis) is plotted against the percentage of falsely positive classified M0-cells on the x-axis. The green curve depicts the result from the training set, while the blue curve represents the unseen test cells. Arrows indicate the pivot values (derived from the training set) used for clustering (see fig.5).

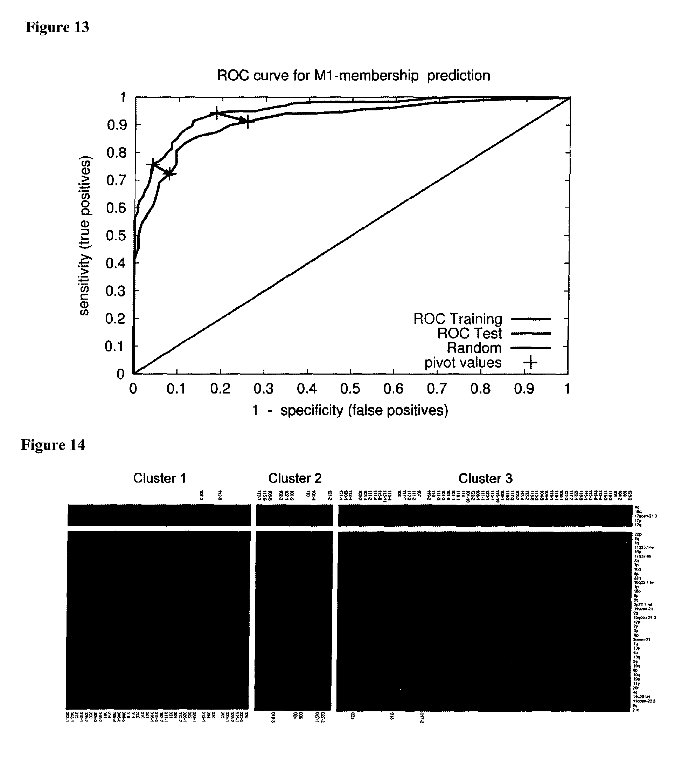

**Figure 14 Identification of metastatic and non-metastatic genotypes**
   All cells were grouped on the basis of their probability to be isolated from a metastatic patient. The pivot values from fig. 4 defined the borders of the three clusters. The five cluster-defining regions are shown in the order of the amount of information they provided to the classification (8q > 18q > 17qcen-21.3 > 17p > 12q). All other genomic regions are ranked according to the feature ranking analysis (see text and supplemental information). Gains and losses are indicated as green and red squares respectively, balanced chromosomal regions are in black color. Chromosomal regions are shown on the right side of each cluster. The clinical stage of the patient is given for each cell below the figure with M0 and M1 for cells from non-metastatic or metastatic patients, respectively.

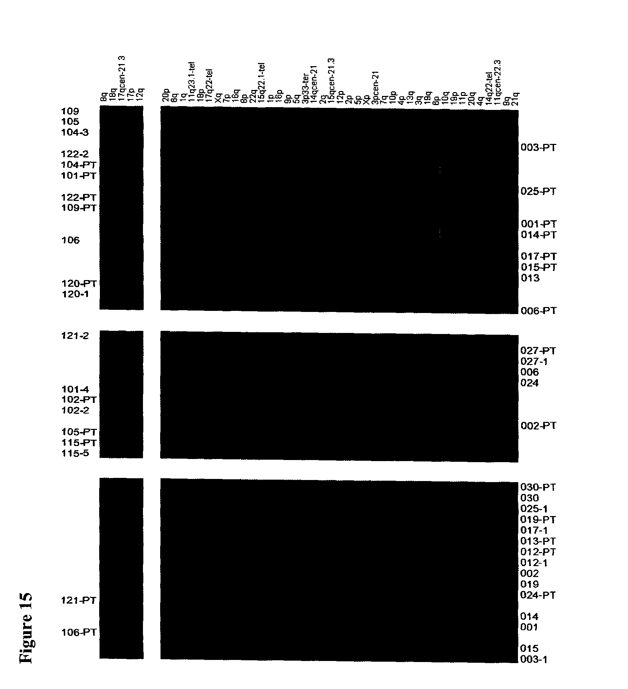

**Figure 15 Comparison of disseminated tumor cells and their matched primary tumors**
   Twenty-four primary tumors and their descendent tumor cells were grouped in three clusters using the same classifier and probability thresholds as in figure 5. Identifiers of metastastic patients are depicted above the clusters, identifiers of non-metastatic patients below. Brackets exemplify the genomic distance between primary tumors and their disseminated cells. (PT, primary tumor; three digit numbers, patient identifier; sister cells are labeled by an additional number).

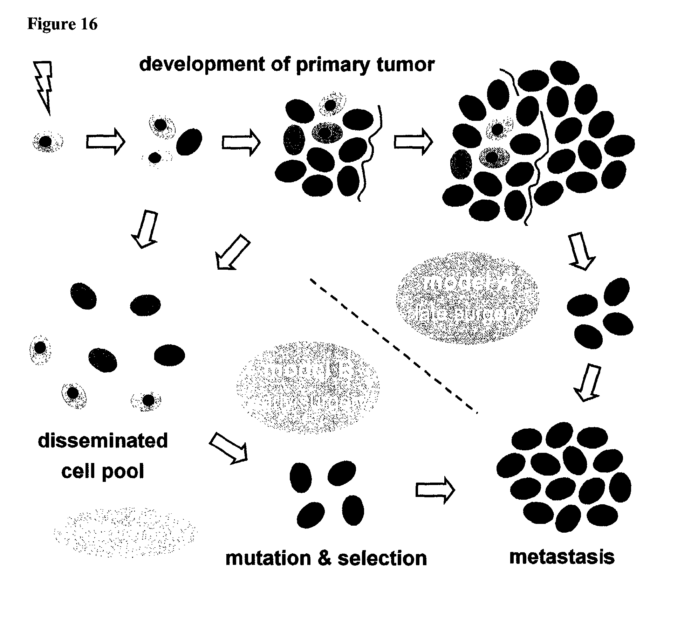

**Figure 16 Model of systemic breast cancer development**
   Tumor cell heterogeneity is depicted by different colors with the blue color indicating cells capable of clonal expansion. Two possible routes are shown that correspond to the two models in the text. Route A represents the linear model of systemic spread that will be most likely occur when the primary tumor is operated lately. Route B depicts the development of disseminated tumor cells into metastasis after a period of tumor dormancy, the time needed for mutation and selection.

In a first embodiment, the framework of the present invention is applied after curative surgery for colorectal cancer for (1) the discovery of clinico-pathological characteristics identifying high-risk patients, (2) the prediction of the development of distant metastases, and (3) the prediction of survival. Furthermore, stage-specific predictions were performed revealing high-risk groups in early tumor stages (AJCC/UICC stages I and II).

### Data and Methods

### Patient data.

The database contains 2931 consecutive cases of colorectal cancer patients diagnosed over 20 years (1978-1998) at the Department of Surgery, University of Erlangen (Germany). After exclusion of AJCC/UICC stage IV and those having residual tumor after surgery (R1 or R2 resections), the remaining set comprises 2403 patients. Table 1 provides a summary of the clinical data for these patients.

**Table 1.**

| Summary of patient data | | | | | | | | |
|---|---|---|---|---|---|---|---|---|
| **AJCC/UICC stage** | **Percent*** | **Survival**^{**§**} | **Metastases**^{**$**} | | **Deaths after year (%)** | | | |
| | | | | **1** | **2** | **3** | **4** | **5** |
| I (n=645) | 26.8 | 126.2 | 7.9 | 2.0 | 4.7 | 7.2 | 10.2 | 12.6 |
| II (n=831) | 34.6 | 117.5 | 16.0 | 1.5 | 5.9 | 10.2 | 14.1 | 17.0 |
| III (n=927) | 38.6 | 82.4 | 37.4 | 6.6 | 16.6 | 23.5 | 28.5 | 32.0 |
| Total (n=2403) | 100 | 106.3 | 22.1 | 3.6 | 10.0 | 15.1 | 19.4 | 22.6 |

| | | | | | | | | |
|---|---|---|---|---|---|---|---|---|
| *Percentage of all patients | | | | | | | | |
| ^{§}Mean survival in months | | | | | | | | |
| ^{$}Percent of patients developing distant metastases | | | | | | | | |

### Statistical Analysis

To provide a realistic application scenario for an expert system for clinical outcome prediction, the data set was divided into training data (Jan. 1978 to Dec. 1982, n=801) and a long test period (Jan. 1983 to Jan. 1994, n=1602). The target variables were (i) the overall survival rate after N years (N=1-5), and (ii) the occurrence of distant metastases. The statistical analysis strategy comprised the following steps as schematized in Figure 1:
(1) *Data preprocessing and transformation:* Clinical variables usually contain symbolic values, e.g., the operation method or the type of post-operative complication. To avoid artificial non-linearities induced by encoding these variables as numbered categories, we transformed the values of each symbolic variable into class-conditional probabilities^{a}. The 124 patient specific clinical parameters were divided into groups of *pre-operative, post-operative,* and *follow-up* attributes^{b}. Follow-up parameters were not used for survival and metastasis prediction.
(2) *Dimensionality of the input vector and variable selection:* The number of parameters in clinical outcome prediction should be kept as small as possible, since the number of variables reflects the dimensionality of the search space for analytical models. Hence, computation of the most important parameters (feature subset selection) for constructing a prediction model is a crucial preprocessing step (a) for avoiding the empty space phenomenon when estimating non-linear functions ^{15,16}, (b) for interpretation of the constructed classifiers, and (c) for increasing the robustness and performance in practical systems. We restricted the number of variables to at most ten^{c}, which were selected from the whole set by a forward selection procedure based on mutual information ¹⁷. In a first step, all variables not having an information content above random noise were discarded (Fig. 8). From the reduced set the variable with maximal mutual information with the target was selected. In the subsequent steps always one additional variable was chosen (Fig. 9). Ten variables were chosen in this way and ranked in the order they were selected.
*(3) Learning from empirical data:* Classifiers were constructed for survival and distant metastases prognosis. The target variables of the training patterns were therefore coded as 1 for positive instances, i.e. the patient develops distant metastases or respectively dies within N years, and 0 otherwise. Artifical neural networks (ANN) were trained using the Bayesian evidence framework ^{16,18}.^{d}
(4) *Survival analysis:* The Kaplan-Meier product limit estimator was used to compare distributions of survival times in different risk groups ¹⁹. For computation of significance levels the Mantel-Haenszel test was performed ²⁰.
*(5) Graphical representation of results and grading risk:* The results obtained from the prediction models on unseen test data were evaluated by receiver operating characteristic (ROC) curves ^{21e}. To evaluate the accuracy of different prognostic models the difference between the respective ROC curves was tested by the Kolmogorov-Smirnoff-test ²².

^{a} 'Class-conditional' in this respect refers to the positive case of either developing distant metastases or dying within N years. Thus, the frequency of a certain value of a variable within the positive class is calculated and the original symbolic value is replaced by its frequency (See Fig. 6).
^{b} See supplementary material (Supp. Tab. I) for a complete list of all 124 parameters.
^{c} See Fig. 7.
^{d} This method applies a cross-entropy error function combined with a weight decay regularizer ^{16,17}. This combined error function was minimized with the Rₚᵣₒₚ algorithm regularizer ²⁵. The network topology was optimized by selecting the models with maximal posterior probability as computed by the Bayesian approach. For example, the optimized classifier for prediction of distant metastases was a single layer network with seven input variables.
^{e} A ROC curve is a graphical technique used to compare models according to their ability to select samples of test cases that have a high proportion of positive instances. The proportion of true positives within the total number of positive cases (sensitivity) is plotted against the proportion of false positives within the total number of negative cases.

### Results

Mean survival was 109 months. To demonstrate the impact of the selected variables for survival prediction, statistical tests on the distribution of survival times between two subgroups of patients discriminated by one or more of these variables were performed. For example, Kaplan-Meier survival curve is given for all AJCC/UICC III patients: roughly 55% survived longer than 5 years (Fig. 2a). When tumors with microscopic venous infiltration were selected, only 45% patients survived the same time period, so that two subsets of patients with different outcome could be split (Fig. 2b). The percentage of survivors was further reduced to 35% when selecting patients who additionally showed low differentiated tumors (Fig. 2c). This process was continued to profile an individual risk. Evidently, in this example, an appropriate variable selection was crucial for survival prediction.

**TAble 2a.**

| Ranked list of selected variables used for metastases prediction (all stages) | | | |
|---|---|---|---|
| **Rank** | **Variable** | **Rank as single var.** | **Description** |
| 1 | AJCC/UICC stage | 1 | Stage I, II, III |
| 2 | Surgical technique | 9 | Different types of surgical procedures |
| 3 | Age | 25 | Age |
| 4 | Degree of differentiation | 13 | Good, moderately, and poorly differentiated. |
| 5 | Depth of tumor infiltration | 3 | Infiltration into submucosa, muscularis propria, subserosa, and beyond serosa |
| 6 | Post-operative complications | 11 | Many possible complications |
| 7 | Venous vessel invasion | 7 | Presence of microscopic vessel infiltration |
| 8 | Localisation of tumor | 21 | Tumor site along the colon and rectum |
| 9 | Lymphnodes metastases at superior rectal artery | 5 | Presence of central positive lymphnodes |
| 10 | Tumorigenesis | 17 | Different types of adenomas, high-risk diseases, and radiation. |

Twenty-one percent of patients developed distant metastases. To select the most informative variables for metastasis prediction, features were ranked with a forward selection procedure based on mutual information as described above. The most informative variable was the AJCC/UICC stage - a tumor-related factor, followed by the type of surgical procedure - a treatment-related factor, and by the patient's age - a patient-related factor. The most informative combination of variables is listed in Table 2 a. Note that many of the high-ranked variables would be ranked much lower if the rank was assigned independently to each individual variable. The selection procedure added variables to the combination that had a low information content by themselves, but increased the information content strongly when used in combination with other variables. This selection process allowed to reduce the number of variables by one order of magnitude (10x) without loss of information - an important feature for mining large data series.

When the model training for prediction of high-risk patients was based on the entire population of patients, the classifier readily identified high-risk patients in stage III (Fig. 2 a to c), whereas high-risk patients in stage I and II were mostly overseen (data not shown). To overcome this limitation, the patients were split into different groups according to their respective AJCC/UICC stage. Applying the feature subset selection procedure as defined above on the individual patient groups revealed a different list of most informative variables for survival prediction of stage I and II groups (Table 2b and 2c). The classification models trained on these different set of variables allowed a much more accurate prediction of high-risk patients for stage I and II (Fig. 3a and 3b), but also to distinguish between high- and low-risk patients within stage III (Fig. 3c). Notably, only by this divide-and-conquer approach it was possible to select patients from stages I and II that have a significantly higher risk of early death^{f}.

**Table 2b.**

| Selected variables used for metastases prediction (AJCC/UICC stage I) | | |
|---|---|---|
| **Mutual Rank** | **Single Rank** | **Description** |
| 1 | 1 | Age |
| 2 | 2 | Operation method |
| 3 | 17 | Infiltration depth |
| 4 | 43 | Lymphnodes metastases at inferior mesenteric artery |
| 5 | 15 | Macroscopic tumor type |
| 6 | 23 | Tumorigenesis |
| 7 | 47 | CEA level (in blood) |
| 8 | 22 | Hypoplastic polyp |
| 9 | 16 | Insufficiency of anastomosis |
| 10 | 41 | Tubular adenoma |

**Table 2c.**

| Selected variables used for metastases prediction (AJCC/UICC stage II) | | |
|---|---|---|
| **Mutual Rank** | **Single Rank** | **Description** |
| 1 | 1 | Operation method |
| 2 | 3 | Invasion of venous vessels |
| 3 | 15 | Age |
| 4 | 27 | Lymphnodes metastases at superior rectal artery |
| 5 | 48 | Gender |
| 6 | 28 | Tumor localisation within the rectum |
| 7 | 9 | Tumor size |
| 8 | 3 | Number of examined lymphnodes |
| 9 | 42 | Grading |
| 10 | 44 | Therapy of worst complication |

^{f} Values obtained in Fig. 2c (combination of two risk parameters) only beat those obtained using classify-it (Fig. 3a), because of the larger number of patients included in the group (180 vs. 108).

In addition to the classification strategy ("classify-it"), a multivariate logistic regression was applied for comparison with state-of-the-art statistic approaches (Fig. 4). For logistic regression, the ten features having the highest correlation with the target variable are used. Notably, this set correlates well with previously published predictive parameters ^{13,14}. Figures 4 and 5 shows that classify-it outperforms the logistic regression for predicting distant metastases as well as survival. ROC-analysis further allowed to adjust the sensitivity of a decision system for assigning risk grades to patients. Table 3a and b shows the number of patients classified correctly by classify-it at different sensitivity levels, as compared to the state-of-the-art treatment decision (all stage III patients treated, i.e. 68.3% sensitivity) and for the logistic regression classifier, respectively. Compared to state-of-the-art treatment decision, classify-it is capable of classifying the same number of patients at risk while reducing the number of false positives by more than 12%. Furthermore, within each stage group the mean survival time clearly shows that the classified patients are at risk of dying early, regardless whether or not distant metastases were diagnosed for these patients.

### Discussion

As therapeutic options improve and broaden, oncologists will need to determine which adjuvant approaches are optimal for the individual patient with colorectal cancer, as well as for society in general. A variety of studies have aimed at the development of personalized cancer profilers for a more precise therapy recommendation and risk assessment for individual cancer patients.

Indeed, with the advent of novel molecular diagnostic tests in combination with intelligent analysis systems, a better understanding of carcinogenesis and thus improved patients' therapy can be expected. However, a variety of molecular studies conducted in the field of colorectal cancer have produced conflicting results on the correlation of various molecular markers and the outcome ¹⁰. Obviously, patient- and treatment-related parameters such as the effect of age, sex, tumor site or the quality of surgery are influencing outcome beside tumor-related, molecular factors .

By developing a novel data-mining framework embedded into a decision-support system, the importance of such patient- related variables is unraveled, such as age and gender, and of treatment-related variables such as kind of surgical procedure and possible postoperative complications. This framework is applied to a data set comprising 124 different variables collected from approximately 2400 CRC patients over more than 16 years. As a result, it was possible to predict with an unrivaled accuracy patient survival and distant metastases. Notably, the patient-specific risk and survival prognosis was much more accurate than a risk assignment based on AJCC/UICC tumor staging only. This is the first study to our knowledge that successfully demonstrates a selection of high-risk patients within each stage of colorectal cancer, in particular in early stages, using artificial intelligence.

The automated selection of the most informative variables significantly improved the prediction of distant metastases development and survival prognosis compared to standard methods. For example, in the present study nearly 55% of patients in AJCC/UICC stage III survived 5 years. This figure is reduced by 10% when a venous invasion was observed, and further 10% in poorly differentiated tumors. For AJCC/UICC stage I and II patients, patient-specific risk profiles based on ROC-analysis could be inferred that could now be used for selecting high-risk patients and thus for indicating adjuvant therapy.

It might first appear puzzling that we did not split our analysis for colon and rectal cancer. This was not necessary because of the very nature of the data mining procedure, that was obviously able to select such variables as the type of surgical procedure and tumor localization, exhibiting high mutual information with the occurrence of metachronous metastases.

Indeed, the present study confirm the importance of "traditional" clinical variables for predicting patient outcome, as opposed to "molecular" data. The present system relies on two different frames of reference : the first could be qualified an "external" frame of reference, provided by past experience in similar cases. The second is an "internal" frame of reference, provided by the previous course of the tumor in the individual case, in particular by the stage of disease and the therapy applied. However, there arc good reasons to speculate that the prognostic question will only be fully addressed on a molecular level. In the near future, molecular screening technologies such as gene or protein expression microarrays will enter the pathology laboratories and complement the current tumor grading technique models ²³. Of course, it is now intended to successively integrate such molecular markers or patterns of disease into the predictive system of the current invention. Since groups of patients with similar prognosis can already be defined, this system can also be applied for validating molecular markers or patterns associated with such outcomes as survival and metastasis, for example.

External validation procedures are a prerequisite for any decision-support system before implementation into clinical practice. The final proof needs to come from randomized clinical studies showing that adjuvant therapy in these patients has an impact on clinically relevant endpoints. Size sample of such future studies will be reduced by applying the present decision-support system to the stratification of patients with a high/low risk of developing metastases. Since the parameters used for building our classification system are based on recognized standards such as AJCC/UICC staging, the acceptance of such a decision-support system by the oncology community and regulatory authorities might be facilitated. In contrast to previous studies ^{13,14} where metastatic patients (AJCC/UICC stage IV) were included, a realistic scenario for a decision-support system in routine application was simulated by selecting only patients after curative surgery. Further, the system was trained on the first third of the data set and validated it with the remaining patients diagnosed in the following 12 years. Even under these difficult conditions, the decision-support system proved its ability for making individual survival prognosis and for assigning a gradual risk of developing distant metastases, thus indicating an adjuvant therapy, for a new patient.

The long follow-up available also points out that tumor recurrences in CRC should be considered over more than 5 years, in particular for early tumor stages. The importance of the history of disease in a particular patient, for predicting outcome in CRC was also nicely demonstrated. To address the question whether pre-operative, post-operative and follow-up variables differ in impact on survival time prediction, three different classifiers were trained based on the three individual variable groups (pre-, post-operative and follow-up) for 1, 2, 3, 4 and 5 year death prediction (data not shown). While there is only a minor performance difference for selecting high-risk patients for 1-year death prediction when using post-operative or follow-up variables, for 5-year death prediction there is a significant difference (p < 0.001) for the models trained on pre-operative and post-operative variables, respectively. The impact of pre-operative variables on survival dramatically decreases over time, whereas the impact of post-operative and follow-up variables gradually increases.

The most widely accepted strategy for adjuvant therapy in resected colorectal cancer (no adjuvant chemotherapy in stages I and II, systematic chemotherapy in stage III, follow-up for both stage II and III, with some differences between USA and Europe) might no longer be considered the reference strategy because of its high cost and its comparatively low efficacy ²⁴. The present tool is an essential first step towards a more accurate identification of high-risk patients in all CRC stages. In particular, it allows to better select the early-stage patients (AJCC/UICC stages I and II) who will benefit of adjuvant chemotherapy.

Finally, we strongly believe that, upon a prognosis generated by any decision-support system, the oncologist will still have to decide together with his patient on an individual therapy. Decision-support systems are designed to assist oncologists in their therapeutic decisions, not to replace them.

**Table 3a.**

| Prediction of distant metastases: sensitivity, specificity and predictive values for different classifiers. | | | | |
|---|---|---|---|---|
| | Classify-IT (i) | Classify-IT (ii) | Standard# | Logistic regression |
| Sensitivity [%] | 58.1 | 68.3 | 68.3 | 68.3 |
| | | | | |
| Specificity [%] | 78.2 | 68.2 | 67.4 | 63.2 |
| | | | | |
| True positives | 207 | 243 | 243 | 243 |
| in stage I/ II/ III | 5/ 22/ 180 | 2/28/213 | 0/ 0/ 243 | 0/ 35/ 208 |
| | | | | |
| False positives | 272 | 358 | 407 | 459 |
| in stage I/ II/ III | 31/61/180 | 5/ 84/ 269 | 0/ 0/ 407 | 3/ 111/ 345 |
| | | | | |
| PPV | 43.2 | 40.4 | 37.4 | 34.6 |
| in stage I/ II/ III | 14/ 27/ 50 | 29/ 25/ 43 | -/ -/ 37 | 0/ 24/ 38 |
| | | | | |
| NPV | 86.7 | 88.7 | 88.1 | 87.4 |
| in stage I/ II/ III | 94/ 86/ 76 | 94/ 87/ 82 | 93/ 84/ - | 93/ 87/ 64 |
| (i) and (ii) : classify-it calculations using two different sensitivity levels # Standard : UICC stage III ("risk") vs. stage I&II ("non-risk") patients PPV : positive predictive value; NPV : negative predictive value. | | | | |

**Table 3b.**

| Prediction of survival : mean survival times (MST) for different classifiers^{g}. | | | | |
|---|---|---|---|---|
| | Classify-IT (i) | Classify-IT (ii) | Standard# | Logistic regression |
| all | 74/102* | 77/104 | 78/105 | 80/105 |
| stage I | 95/109 | 57/108 | -/107 | 87/107 |
| stage II | 94/105 | 93/105 | -/103 | 89/108 |
| stage III | 68/90 | 72/91 | 78/- | 77/80 |

| | | | | |
|---|---|---|---|---|
| ^{*} MST for risk/non-risk patients. (i) and (ii) : classify-it calculations using two different sensitivity levels. # Standard : UICC stage III ("risk") vs. stage I&II ("non-risk") patients. | | | | |
| ^{g} MST are computed only for patients in test set. Hence, the MST shown here are lower than the MST for all patients (see Table 1). | | | | |

In the following, a second embodiment of the present invention will be described.

Until today the nature of minimal residual cancer, the critical tumor rest left behind after surgical resection, has remained elusive because the extremely rare disseminated tumor cells at this stage of disease have defied any closer examination. The lack of knowledge about these cells is contrasted by the almost routine administration of adjuvant therapies to early-stage breast cancer patients. The present study on the genetic evolution of disseminated tumor cells provides new insight into the process, how generalized metastasis develops and how this can be exploited for an early risk assessment of an individual patient. Contrary to the current paradigm of a linear relationship between the primary tumor and its metastases, the data presented here indicate that the seeds of metastasis develop to a large degree independently at a distant site, particularly in patients whose tumors have been operated early. Therefore, the direct analysis of the disseminated tumor cells rather than the extrapolation from the excised primary tumor is more informative to unravel novel mechanisms and targets for adjuvant therapies and specify the need of an individual patient for timely systemic treatment.

According to the prevailing paradigm, cancer progresses in a linear fashion after a model that was derived from the adenoma - carcinoma sequence observed in some forms of colorectal cancer (Vogelstein et al., 1988; Vogelstein and Kinzler, 1993). That model describes a stepwise accumulation of multiple mutational events preceding the carcinoma stage, and predicts that certain additional genetic and epigenetic changes are required for progression to generalized disease. Indirect evidence for this hypothesis comes from experimental mouse tumors where cells with metastatic potential can be selected from the primary tumor (Fidler and Kripke, 1977). Furthermore, in these systems alterations and mechanisms were defined that differentiate stationary from metastasizing cells (Clark et al., 2000). In breast cancer, however, cytogenetic analysis has failed so far to identify a gradual acquisition of genomic changes, since in-situ carcinomas and invasive carcinomas displayed very similar chromosomal aberrations (Allred et al., 2001; Aubele et al., 2000; Buerger et al., 1999a; Waldman et al., 2000). Likewise, similar genetic alterations were observed in primary tumors and synchronous regional lymph node metastasis (Nishizaki et al., 1997; Pandis et al., 1998). In contrast, when metachronous, distant metastases were investigated by comparative genomic hybridization, they differed extensively from the corresponding primary tumors (Kuukasjarvi et al., 1997), a finding that contradicts a simple linear descent model. It also prompted us to ask whether in breast cancer, metastases evolve at distant sites quite differently from the primary tumor through independent acquisition of mutations. After resection of a localized primary tumor the founder cells for metastases reside at an ectopic site, defining minimal residual disease. Genomic analysis before ongoing genetic instability blurs the descendence of these precursor cells might therefore reveal the relationship between primary tumor and clinically evident metastasis.

Disseminated tumor cells can be identified in bone marrow with monoclonal antibodies, although at a rather low frequency of 10⁻⁵ to 10⁻⁶ normal nucleated cells. Their detection is based on the intracellular staining of epithelial cytokeratins that are not expressed in the exclusively mesenchymal bone marrow compartment (Schlimok et al., 1987). Interestingly, single cytokeratin-positive cells are not only specifically detected in bone marrow samples of carcinoma patients without clinically evident metastases but, when found, they are also strongly correlated with development of distant metastases and prognosticate a shortened survival of the patient (Braun et al., 2000b; Janni et al., 2001). To answer the question of metastatic progression in breast cancer, the efforts are concentrated on the genomic characterization of single disseminated cancer cells (Klein et al., 1999) as the link between the primary tumor and its later arising metastasis.

### Results

### Isolation of single disseminated tumor cells

Bone marrow was aspirated from 371 unselected breast cancer patients after informed consent was obtained. Of the patients who had no evidence of metastasis 44% of the samples were drawn during surgery, 39% within the next month, and 17% one month to 10 years after surgery of the primary tumor. For immunocytochemical detection antibodies most commonly used are applied that are directed against cytokeratins, the mab CK2 (specific for cytokeratin 18) and the mab A45-B/B3 (recognizing a common epitope on cytokeratin 8, 18 and 19) (Klein, 2000), (Pantel et al., 1994). Ninety-nine patients were found to contain one or more cytokeratin-positive cells in their bone marrow samples (26.7 %; table 4).

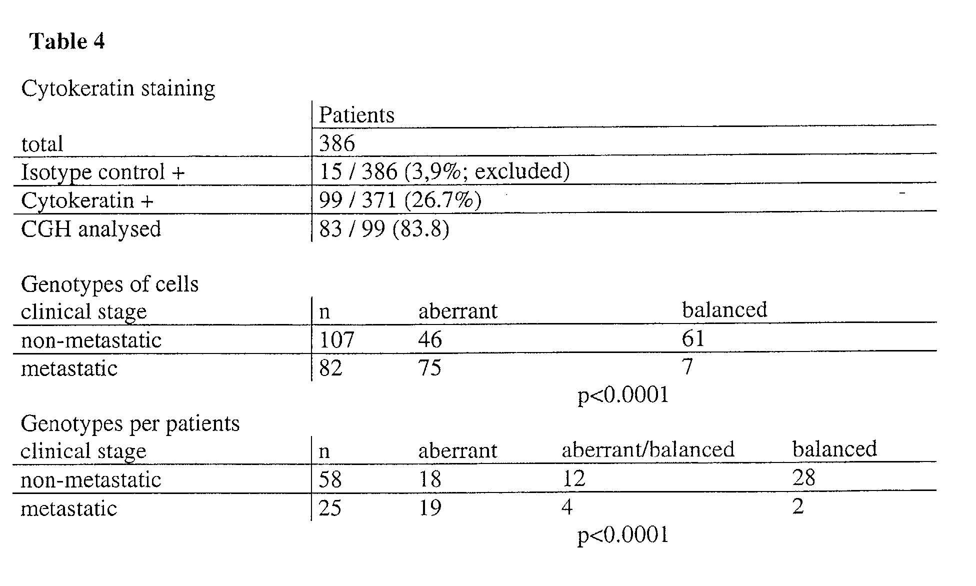

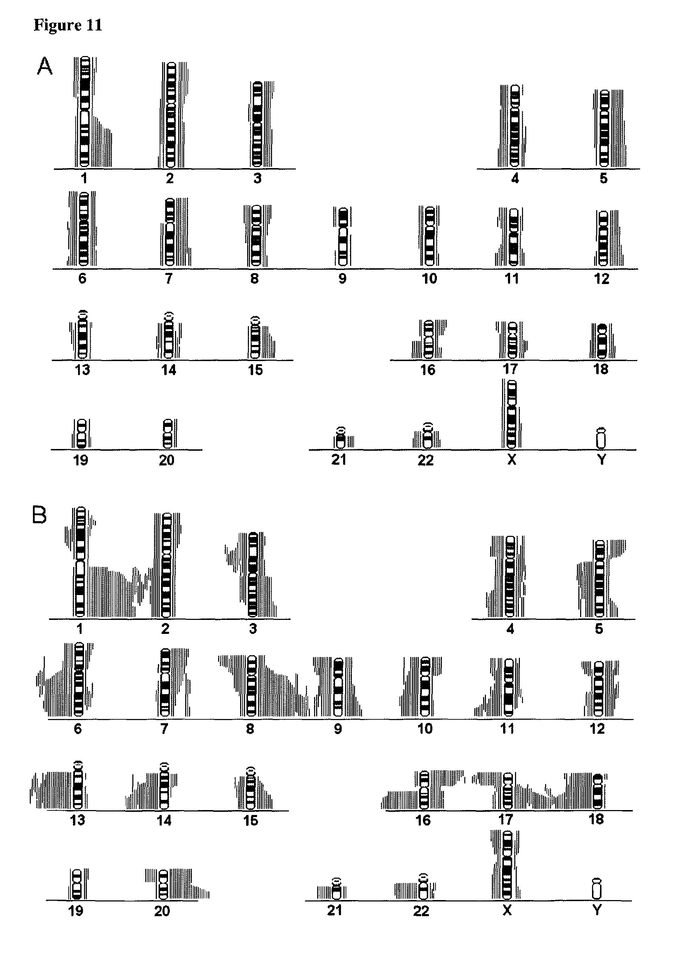

The labeled cells were individually isolated by micromanipulation from the adhesion slides, their genomic DNA was prepared for global amplification as previously described (Klein et al., 1999) and the amplified DNA used for comparative genomic hybridization (CGH) (fig. 10). From 83 of 99 patients (83.8%), single cell CGH could be successfully performed. The status of the patients' disease was postoperatively assessed and they were staged according to the standards of the UICC. Of the 83 patients, 58 patients had no evidence of metastatic disease (UICC stage M0) while 25 presented with manifest metastasis (UICC stage M1) at the time of bone marrow aspiration. In total, 189 cells were analyzed, comprising 107 from patients in stage M0 and 82 from patients in stage M1 cells. Sixty percent of patients for whom clinical information on the metastatic site was available presented with bone metastasis at sites different from the pelvis where the marrow was aspirated, while the remaining patients had their metastases at various other sites such as lung, liver, distant lymph nodes or skin. From 40 patients more than one cell could be isolated and the percentage of these patients with sister cells was higher in M1 collective than in M0 stage (68% vs. 40%; fig. 11).

### Genotypes at dissemination and metastasis

68 cytokeratin-positive cells from 46 patients were found to have no CGH detectable chromosomal aberrations (i.e. balanced CGH profiles; table 4). Chromosomal gains or losses are picked up by CGH if they comprise chromosomal material larger than 10-20 Mb. From 30 patients only cytokeratin-positive cells with balanced CGH-profiles could be isolated while 16 patients had simultaneously CGH-balanced and CGH-unbalanced cells in their bone marrow (table 4). Strikingly, CGH-balanced cells in absence of CGH-unbalanced cells was found in 28 of 58 (48%) M0 patients in contrast to M1 patients where 2 of 25 (8%) displayed such cells (p<0.0001).

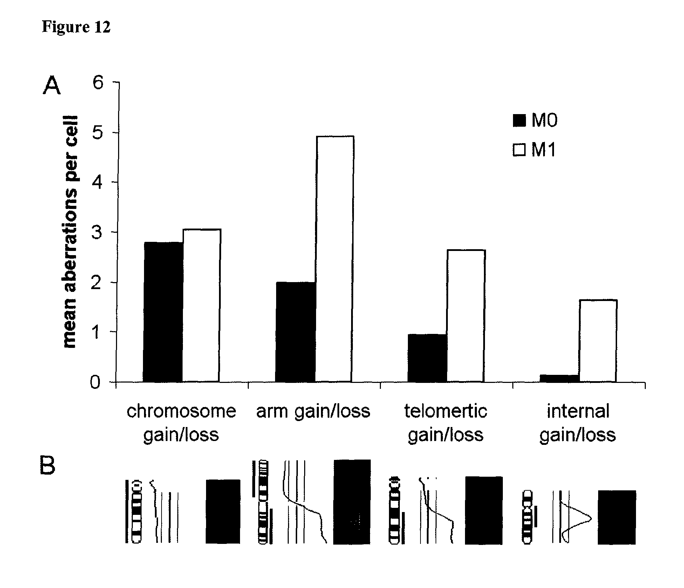

A comparative analysis of 46 cells with chromosomal aberrations from stage M0 patients and 75 cells from stage M1 revealed an increase of the mean number of chromosomal aberrations per cell from 5,9 (± 4,1 SD) to 12,8 (± 5,4 SD) in disease progression (p<0.0001). Figure 12a and 12b provide an overview of the aberrations present in M0 and M1 breast cancer cells. To depict the diversity of karyotypes retrieved from disseminated tumor cells and to avoid a bias in favor of patients with multiple cells, sister cells with identical CGH-profiles were excluded. As can be seen, certain chromosomal aberrations (present in 40% of the analyzed cells or more) are much more frequent in M1-cells, such as amplification of 1q, 8q, 16p, 17q, 20q and loss of 6q, 13q, 14q, 16q and 18q. However, there are some chromosomal regions such as amplifications of chromosome 5cen-5q23.3 (20% in M0 vs. 4,2% in M1 cells) and 18q (12,5% in M0 vs. 2,1% in M1 cells) that are apparently more often affected in M0-cells. Taken together, the genomes of disseminated cells that were isolated from the two patient groups differed largely.

### Types of chromosomal aberrations

As can be seen from the histograms in Figures 12a and 12b, patients in M0 differ from patients in stage M1 with regards to the affected chromosomes and to the total number of changes. It also suggests that the two groups display different types of structural changes because aberrations in M1 cells appear more regionally focussed and confined than those seen in M0 cells. It was therefore analyzed whether specific types of aberrations were associated with metastatic progression and which chromosomal changes are present closer to the time point of dissemination. The CGH data were evaluated for numerical genomic aberrations affecting whole chromosomes, chromosome arms, defined intrachromosomal and telomeric regions. Interestingly, cells from M0 breast cancer patients had mostly gained or lost whole chromosomes (fig. 13), while the genomes of M1 cells showed an significant increase of all changes involving chromosome breaks (p < 0.0001).

### Stage-defining genomic aberrations

Given the impressive difference between M0 and M1 cells it was then investigated whether distinct genomic aberrations are associated with the clinical stage of the patients. The goal was to define a subset of genomic changes that contain the information whether a disseminated tumor cell was isolated from a patient in M0 or M1 stage of disease. To do so, a feature ranking analysis is performed for chromosomal aberrations to determine the functional relationship between the aberrations and clinically evident metastatic disease. The ranking was achieved by a forward selection procedure based on mutual information between feature and target [Ragg, 2002]. Briefly, 46 genomic loci were selected as features - mostly comprising chromosome arms unless they were more clearly defined on the basis of the aberrations shown in figure 12. Secondly, the presence of clinically evident metastasis was defined as target and ranked all 46 genomic loci according to the amount of information that was provided by their status (gain, loss or unchanged) for the identification of a cell that was isolated from a M1 patient. . The maximal mutual information of a single feature was provided by the status of 8q. In each subsequent step the algorithm determined those loci that added highest information content of the remaining to the already selected features.

Thereafter, several classifiers were trained to select the subset of loci that predict metastatic disease with highest accuracy. As classifiers simple multivariate logistic functions were used (which correspond to single layer neural networks). Further, a final subset of features was selected that corresponds to the determined model with maximal model evidence . These features were 8q, 18q, 17qcen-21.3, 17p and 12q.

Several prediction models were then compared for their correct classification of test samples that had not been seen by the classifier during training by applying a 9-fold cross-validation procedure and by analyzing the averaged results with ROC (receiver operating characteristic) curves [Empson 2001]. The percentage of truly positive classified M1-cells (y-axis) is plotted against the percentage of falsely positive classified M0-cells on the x-axis (fig. 14). The prediction capability of a model is depicted by the steepness of the slope, while random guessing corresponds to a diagonal line. Figure 13 shows the predictions of the classifier that used the five selected aberrations, with highest sensitivity and the lowest number of false positives. To classify the cells into three clusters the pivot probability values were derived from the averaged ROC-curve on the training data (fig. 14).

Firstly, all cells with a probability below 0.22 of being isolated from a patient with clinically evident metastasis where assigned to cluster 1. Secondly, all cells with a probability above 0.75 were grouped in cluster 3. All other cases formed cluster 2, since the classifier could not assign them with a high probability to either cluster 1 or cluster 3. Figure 6 shows the result of this cluster analysis for all cells. While cluster 1 contained 37 M0 cells and 2 M1 cells, cluster 3 comprised 57 M1 cells and 3 cells from patients in minimal residual disease. Five cells from M0 and nine cells from M1 patients were grouped into cluster 2.

The cluster analysis allows to draw several conclusions. Firstly, cluster 3 comprised cells from all but one M1 patients, because the two M1 cells detected in cluster 1 had sister cells in cluster 3. Only the cells from M1 patient #102 were placed in cluster 2 with no other cell from this patient being classified in cluster 3. Secondly, cluster 3 comprised M1 cells regardless of the site of overt metastasis. Consequently, the subset of aberrations defining cluster 3 seems to be predictive of metastasis anywhere in the body and M1 cells apparently are representatives of metastasis. Thirdly, the isolated amplification of 8q or 17qcen-21.3 and the isolated loss of 18q (and to a lesser degree loss of 17p) were sufficient for the assignment to cluster 3 (= cluster-defining mutations). In contrast the sole opposite mutation (i.e., loss of 8q / 17qcen-21.3 or amplification of 18q / 17p) or the complete lack of any of the cluster-defining mutations was sufficient for cluster 1 assignment. Isolated loss of 17p lead either to the assignment to cluster 3 or cluster 2 depending on other mutations. Loss 12q or unchanged 12q increases the probability to be a M1 cell while amplification of 12q decreases the probability. Finally, Cluster 2 includes mostly cells, which have a mixture of the cluster-defining mutations and their opposite mutations.

### Comparison of disseminated cells with their primary tumor

Assuming a linear model of tumor progression one would expect most primary tumors of the collective to harbor less often cluster-defining mutations than the disseminated tumor cells from M0 patients or to display them with similar frequency. Amplification of 8q was found in 10%, loss of 18q in 13%, 17q gain in 13%, 17p loss in 17% of cells isolated from patients with minimal residual disease. However, amplification of 8q, 17qcen-21.3 and loss of 18q, 17p, has been found in 50%, 25%, 35% and 35%, respectively, and belong to the rather frequent aberrations in primary breast cancers (Buerger et al., 1999b; Nishizaki et al., 1997; Roylance et al., 1999; Tirkkonen et al., 1998; Waldman et al., 2000).

To resolve this obvious discrepancy between published data and the M0 cells 27 primary tumors of the collective were analyzed. After pathological examination, areas with malignant morphology were laser microdissected and the DNA was prepared as recently published applying the same global amplification method as for the single cells. Three tumors belonged to the group of patients of whom only CGH-balanced cells had been isolated from bone marrow. These tumors harbored multiple chromosomal aberrations (data not shown). Of the remaining tumors, amplification of 8q was found in 50%, loss of 18q in 18%, amplification of 17qcen-21.3 in 57% and 17p loss in 21% and the complete CGH profiles were then analyzed by the described classifier together with their disseminated tumor cells . According to a linear model, the primary tumors should be mostly grouped in cluster 1. However, only 7 of 24 tested primary tumors were assigned to cluster 1 (fig. 16). Moreover, when comparing the overall similarity between primary tumors and M0 / M1 cells by computing the differences of the assigned probability values as a measure for the genomic distance, it was found that the primary tumors and M0 cells were significantly less related (p < 0.002) than the primaries and M1 cells. Taken together, most M0 cells disseminate from the primary tumor before the primary tumor has acquired the cluster-defining mutations, while M1 cells and primary tumors both harbor those aberrations.

### Diagnostic implications

Tumor cells that are isolated from the bone marrow have already taken an important hurdle towards metastasis, i.e. the invasion of an ectopic organ, and could confer diagnostic information additional to that provided by the primary tumor. The predictive power of genomic aberrations of single disseminated breast cancer cells was therefore compared with classical prognostic markers such as tumor size, lymph node involvement, estrogen and progesteron receptor status and the her2 positivity, here summarized as clinical routine variables. Because the analysis was performed on a subset of cases (M0, n = 29; M1, n = 10) for which the clinical routine variables were available a leave-one-out procedure was applied. The predictive power of the two classifiers, i.e the clinical routine variables versus the cluster-defining mutations of disseminated tumor cells was then compared . In contrast to the three clusters above, the classification threshold of 0.5 was chosen, i.e. the probability of larger or less than 50% of a cell of being derived from a clinically metastasized patient. From some patients several cells were isolated for which different probabilities of being isolated from a metastatic patient had been calculated by the classifier. Therefore, the predictive power of disseminated tumor cells was determined twice, using either those cells with the highest or with the lowest probability, which is reflected in the best and worst predictive power, respectively (Table 5). Thereby, the range of prediction power due to a sampling error can be determined.

**Table 5:**

| Comparison between the predictive power of the best combination of clinical routine variables (i.e. the T and N stage) and of the classification derived from the genomic aberrations of disseminated tumor cells. | | | | | |
|---|---|---|---|---|---|
| | Direct comparison for 39 cases | | | All cases | |
| | Clinical routine variables | Prediction from disseminated cells | | | |
| | | best | worst | best | worst |
| Accuracy | 69.2% | 92.3% | 87.2% | 94.3% | 84.9% |
| PPV | 25% | 81.8% | 72.7% | 91.7% | 85.7% |
| NPV | 74.3% | 96.4% | 92.8% | 96.5% | 84.4% |
| Sensitivity | 10.0% | 90.0% | 80.0% | 95.6% | 78.3% |
| Specificity | 89.7% | 93.1% | 89.6% | 93.3% | 90.0% |

Using the five cluster-defining aberrations as input features the classification performance reached 87-92% (worst-best prediction) of correctly classified patients compared to only 69% when the best combination of clinical variables, which in the collective was the T and N stage, were used as input (p<0.002). The positive and negative predictive values (PPV and NPV, respectively) of both classifiers specify the better prediction by disseminated cells. While the metastatic disease of only one of 10 patients could be predicted by the clinical routine parameters (with three patients being falsely positive classified), nine from 10 M1 patients were correctly predicted based on the five aberrations with much higher sensitivity (Table 5). On the other hand, absence of clinical metastasis was correctly identified in 74% by clinical parameters and in 93-96% by the classifier based on the genomic imbalances of disseminated tumor cells with almost identical specificity. To test whether the high predictive power seen for the disseminated tumor was biased by the 39 patients, it was calculated for all 53 cases that had cells with genomic aberrations. Only marginal differences were observed between the two groups (Table 5). Thus, the presence of generalized disease in a breast cancer patient can be predicted significantly better by the genomic analysis of a *single* disseminated cancer cell isolated from her bone marrow than by analysis of her primary tumor, the local lymph node status or a combination of several clinical routine variables. Moreover, while the status of the primary tumor is only assessed once after surgery, repeated bone marrow aspirations and genomic analysis of isolated tumor cells are feasible during follow-up allowing a continuous monitoring of the patient.

### Discussion

On the genomic abnormalities of single disseminated cancer cells that were isolated from the bone marrow of breast cancer patients who presented in two distinct stages of clinical progression, in minimal residual disease (stage M0) and overt clinical metastasis (stage M1) is reported. The specificity of epithelial cytokeratin was exploited as marker for disseminated carcinoma cells in bone marrow and a recently developed technique that enables whole genome amplification of a single cell (Klein et al., 1999). This method allows the amplification of any single copy sequence as defined by two Mse I restriction sites with 90% reliability (Klein et al., 1999), and Klein, unpublished data.

Surprisingly the number, kind and pattern of CGH-abnormalities of M0 cells differed largely from M1 cells as well as from primary tumors. The findings challenge widely held views on the timing of dissemination and metastasis formation.The data look even more irritating when one considers the well established fact that the finding of cytokeratin-positive cells per se in bone marrow of M0 patients is highly predictive for a bad prognosis (Braun et al., 2000b; Jauch et al., 1996; Lindemann et al., 1992; Pantel et al., 1996; Weckermann et al., 2001); thus one cannot dismiss these cells as irrelevant.

First of all, M0 cells comprise a genomically very heterogeneous population. From 52% of M0 patients with positive bone marrow finding cytokeratin-positive cells could be isolated without any chromosomal abnormality given the limited resolution of CGH (10 to 20 Mb). Although it cannot positively be proven the malignant nature of each individual cytokeratin-positive cell at the moment, the occurence of cytokeratin-positive cells without gross genomic aberrations is relatively common in minimal residual cancer but is rare in overt metastatic disease, i.e. 89,7% of the cells were isolated from M0 and 10.3% from M1 patients. Furthermore, cytokeratin-positive cells in patients without epithelial neoplasias are rather rare (< 4%) (Braun et al., 2000b). Consequently, the relatively frequent finding of CGH-balanced, cytokeratin-positive cells strongly suggests that dissemination can precede the acquisition of gross chromosomal aberrations, while metastatic expansion of a disseminated cell may depend on the emergence of chromosomal instability.

Additional support for this interpretation comes from a closer analysis of the different types of genomic aberrations detected by CGH. The majority of chromosomal aberrations present in M0 cells consisted in aneuploidy, while M1 cells showed a dramatic increase of changes involving chromosomal breaks. Among the changes that increased with disease progression were those that most likely result from telonomic instability (Artandi et al., 2000) (fig. 12) because the breakage included the telomeric regions, while gains or losses of whole chromosomes - as seen in M0 cells - are caused by different mechanisms (Pihan and Doxsey, 1999). Interestingly, one current model for malignant transformation and immortalization of normal human mammary cells proposes several restriction points to overcome senescence, which have been termed selection (formerly M0), agonescence (formerly M1) and crisis (formerly M2) for human mammary cells (Romanov et al., 2001). Mammary cells seem to escape selection by inactivation of the Rb pathway and in particular by loss of p16 expression. Post-selection cells display first chromosomal changes and crisis will result in multiple non-reciprocal translocations due to telomere attrition, fusion and chromosome breakage. Reactivation of telomerase stabilizes the genome after crisis, the cell becomes immortal and forms a tumor (DePinho, 2000). The time point of telomerase reactivation has been shown to occur early in breast cancer development and was already found in about 75% of ductal in-situ carcinomas (Hiyama et al., 1996; Poremba et al., 1998; Tsao et al., 1997). It is therefore very tempting to speculate that cytokeratin-positive cells in bone marrow that do not display CGH-abnormalities, correspond to pre-selection cells, while cluster 1 cells being equally common for M0 patients would represent post-selection cells. Finally, post-crisis cells displaying telomeric changes would be indicative for M1 patients (cluster 2 and 3 cells). If so, mammary tumor cells disseminate early in the transformation process and have to go through crisis at a distant site prior to forming a metastasis. Thus, cells would develop into manifest metastases independently from the primary tumor.

When the patterns of genomic aberrations of M0 and M1 cells are compared the most obvious findings are the differences between the sheer number of aberrations and of the genomic regions involved. Furthermore, using machine-learning methods the influence of each chromosomal region was assessed with regard to its power to predict whether a disseminated tumor cell is isolated from a M1 patient. The five chromosomal regions that contributed most information with regard to assignment to cluster 3, the cluster with highest probability values, were 8q, 18q, 17qcen-21.3, 17p and 12q.

Interestingly, cluster 3 comprised M1 cells regardless of the anatomic site of the clinically evident metastasis. These cells come closest to the definition of true micrometastases and obviously represent clinically evident metastasis. A plausible explanation for this finding could be that the five cluster-defining mutations are associated with clonal expansion of a cell forming a metastasis. If so, these mutations might also drive clonal expansion in the primary tumor. Indeed, one or several of the cluster-defining mutations (excluding 12q for its minor role) were found in 84% of primary tumors investigated. Because the cluster-defining mutations were originally extracted from the analysis to differentiate M1 cells from M0 cells, it is evident that primary tumors are significantly more similar to M1 than to M0 cells (p < 0.002).

If the cluster-defining mutations are present in 84% of primary tumors how can one reconcile this with the finding that only 12-25% of patients (depending on whether the CGH-balanced cytokeratin-positive cells are included or not) in the stage of minimal residual disease have disseminated cells harboring these aberrations? At least two scenarios can be imagined. The classical concept (model A) assumes that it is most likely that a cell from the predominant clone of the primary tumor disseminates and forms a metastasis because it already is a post-crisis cell harboring mutations required for clonal expansion and only has to acquire additional mutations necessary for dissemination or ectopic survival (fig.16). Therefore, it is difficult to understand why mostly pre-crisis cells without the cluster-defining mutations are detected at the stage of M0. Although model A can explain why M1 cells are similar to the primary tumor, it cannot explain why M0 are completely different from the primary tumor. In model A, the disseminated cells from M0 patients would represent "unproductive" cancer cells that will not progress. However, the finding of these "unproductive" cytokeratin-positive cells, has strong prognostic impact on the clinical outcome of most human carcinomas (Braun et al., 2000b; Izbicki et al., 1997; Jauch et al., 1996; Lindemann et al., 1992; Pantel et al., 1996; Thorban et al., 2000; Weckermann et al., 2001). It is important to note that all patients of the present study underwent surgery. Therefore, the main source of post-crisis, M1-like cells had been removed prior to bone marrow sampling or within the next hour after the bone marrow was aspirated. Consequently, model A implies that the metastasiogenic cell (post-crisis, M1 like) hides at a site different from bone marrow inaccessible for analysis in the majority of M0 patients. Obviously, this is in contrast to the clinical experience that bone marrow, i.e. skeleton, is by far the preferred site of breast cancer metastasis, and to the fact that cytokeratin-positive cells in bone marrow of breast cancer patients are predictive for the development of skeletal metastasis (Braun et al., 2000b).

The present data suggest a different model (B), where tumor cells frequently disseminate in a "genomically immature" state, before they undergo telonomic instability and suffer major CGH-detectable aberrations (fig. 16). At the distant site they undergo several steps in the transformation process in parallel to the primary tumor, whose major population is selected for local proliferation and expansion displaying a more sessile phenotype. In most cases the evolutionary process at the ectopic location may lag behind the primary tumor formation because the disseminated cells experience various growth constraints. The concept of independent evolution firstly explains why distant metastases often display aberrations that are incompatible with a simple linear relationship to the primary tumor (Kuukasjarvi et al., 1997), and secondly provides a plausible explanation for the concept of tumor dormancy (Demicheli, 2001; Riethmuller and Klein, 2001; Willis, 1952). The latency period, which in breast cancer often lasts several years, sometimes decades, may be equivalent to the time required for the "immaturely" disseminated tumor cells to evolve. This genetic explanation may add to dormancy mechanisms that have been described earlier (Uhr et al., 1997). However, the clinical observation that 0,5-7% of diagnosed cancers are so called "cancer of unknown primary", i.e. metastasis without detectable primary tumor (Abbruzzese et al., 1994), strongly support the hypothesis of an early independent evolution of disseminated cells and primary tumors. One difficulty with model B is, that primary tumors and M1 cells are more closely related than primary tumors and M0 cells. However, this can be resolved by the concept of convergent karyotypic evolution. The finding that M1 cells clustered together in Cluster 3, although they were derived from different patients with clinically diverse metastatic sites illustrates this concept. The recurrent combination of the cluster-defining aberrations is very suggestive of a converging pathway of karyotypic evolution during breast cancer progression. Additional aberrations were very often detected together in M1 cells although they had a minor impact on the classification. Such mutations were loss of 6q, 16q and 13 and gain of 1q and 20q. It has long been suggested that in contrast to the rather stable balanced translocations characteristic of leukemias and lymphomas, the development of most solid malignancies depends on an intricate combination of deletions and amplifications of multiple chromosome segments (Fearon and Vogelstein, 1990; Vogelstein and Kinzler, 1993; Weinberg, 1989). A cytogenetic analysis on more than 3000 solid tumors revealed maps of chromosomal imbalances (Mertens et al., 1997) from which converging pathways of solid tumor evolution could subsequently be retrieved (Hoglund et al., 2001). It is plausible to apply the concept of such a convergent evolution that was derived from tumors of various patients to the different clones of an individual tumor during progression. Consequently, the closer relatedness of metastatic cells and primary tumors reflected by similar patterns of aberrations can be seen as the result of an independent selection process leading to rapidly growing clones.

Both models need not to be mutually exclusive but may apply to different groups of patients with different probabilities. Model A is more likely to describe the metastatic progression of patients with large tumors and locally extensive disease due to the fact that a very aggressive cell will be selected from billions of cells in a rapidly growing tumor at some point of time. Model B will most likely fit to patients with small tumors that are operated early. Here, the cells selected for proliferation in the primary tumor, will less likely represent the potential founders of metastasis. Instead, in these patients early-disseminated cells will have the time to acquire mutations needed for clonal expansion and metastasis formation.

The present study has several implications for the future investigation of breast cancer. First of all, the retrieved genomic aberrations appeared to be more predictive than any other prognostic factor for metastatic disease including lymph node status, estrogen-, progesteron receptor status and her2 positivity or their combination. In essence, the genome of a single disseminated cell apparently predicts with 85-94% accuracy whether a patient has metastatic disease or not. This accuracy has so far never been reached by any marker tested on primary tumors. It can therefore be foreseen that further subclasses within cluster 1 or 2 define the risk of M0 patients to develop metastatic disease. The prime candidates are those M0 patients that already have disseminated cells assorted to cluster 2 and 3. However, this has to be tested and confirmed in prospective longitudinal studies. Most importantly, several authors have shown that disseminated cells can be monitored over prolonged periods of time in individual patients (Braun et al., 2000a; Heiss et al., 1995; Schlimok et al., 1990). The assessment of the genomic "maturity" of disseminated cells during follow-up might thus provide a completely novel basis on which the need of a patient to receive an adjuvant therapy can be determined.

Finally, the striking difference of disseminated tumor cells in the stage of minimal residual disease from their primary tumors and the concept of an independent allopatric evolution favors the direct analysis of disseminated cells over the current practice to base adjuvant therapies on target structures that were derived from primary tumors. To get access to the phenotype of the seed of metastasis a protocol for transcriptome analysis of single disseminated cancer cells (Klein et al., 2002) has recently been developed. A single cell approach directed at genetic and epigenetic changes might be able to reveal a common and essential canon of molecules, on which the harbingers of clinical metastasis depend and that is sufficiently different from normal untransformed cells.

### Methods

### Immunocytochemical Analysis and Micromanipulation

Preparation of bone marrow suspensions was performed as published (Klein et al., 1999). Suspensions of 10⁶ cells in 0,5 ml PBS were placed on adhesion slides for sedimentation (30 min.) (Micromet, Germany) and dried. Detection of cytokeratin positive cells was performed by immunocytochemical staining using mab A45-B/B3 Fab fragment conjugated to alkaline-phosphatase (Micromet, Germany), mab A45-B/B3 APAAP (Micromet, Germany) and mab CK2 APAAP. MOPC 21 antibody (Sigma) served as isotype control. Slides were developed by alkaline posphatase using BCIP/NBT (BioRad) as substrate. Micromanipulation was performed as published (Klein et al., 1999). Tissue sections of paraffin embedded archive material were stained with Haematoxylin and tumor areas were microdissected using the Laser MicroBeam System (P.A.L.M.) .

### Single Cell PCR and Comparative Genomic Hybridization

Single cell PCR and CGH were performed as published in (Klein et al., 1999) with minor modifications. The primer sequences of the adapter were changed to 5'-AGT GGG ATT CCT GCT GTC AGT-3' and 5'-TAA CTG ACA GC dideoxy-3'. The CGH probes were labeled with Digoxigenin-dUTP (tumor DNA) and Biotin-dUTP (reference DNA).

### Feature ranking analysis

Mutual information is the amount of information about the target variable t (here the M1 stage of the patient) that is provided by each of the 46 genomic loci (features). The mutual information of the input vector X and the target variable t measures the degree of stochastic dependence between the two random vectors (Cover and Thomas, 1991). If the mutual information value is high, X carries much information about t. All features are ranked by the amount of mutual information that they contribute to the target in repeated rounds of selection (Ragg et al., 2002).

### Classifier construction and model selection

Using the selected set of features a classifier is built that assigns each cell its probability of being isolated from a M1 patient. Several classifiers with different numbers of input features were trained. The weight vector w of the classifier is determined by using the Bayesian evidence approach (Bishop, 1995; Ragg 2001) to minimize the Cross-Entropy error function. To select the final subset of features the model evidence P(D|Theta) is computed within the Bayesian framework for each classifier, where Theta = (w, h) is the model parameter vector, consisting of the weight vector w and the input structure h of the classifier. The subset of features is selected that corresponds to the classifier with maximal model evidence.

### Cross validation, ROC-Analysis and cluster design

The classifier was 9-fold cross-validated by splitting the data in 9 equal parts and using each part once for testing and the remaining 8 parts for training. Generalization performance was averaged over these 9 cross-validation sets. Furthermore, leave-one-out errors were determined by using one cell for testing and the remaining cells for training. The overall error was then averaged over all single experiments. Receiver operating characteristic (ROC) curves (Empson 2001) confirmed the model selection process. The Kolmogorov-Smirnov (Press et. al, 1992) test was used to test for significant differences between the predictive power of models. Pivot values for the definition of the three clusters were determined on the averaged ROC-curve on the training data (fig. 14) by selecting a first point from the left side where the steepness of the the ROC-curve decreases strongly and the second point from the right side where the steepness increases strongly (fig. 14). Each of these points corresponds to a probability value which is used as pivot value to assign cells from unseen data to clusters.

### Evaluation of clinical routine variables

A classifier based on clinical variables (tumor size, lymph node status, tumor grade, oestrogen / progesteron / her2 receptor status) was built as for the genomic loci of the isolated tumor cells. On the subset of cases for which clinical variables where available the leave-one-out error was computed to determine the predictive power of the various combinations of the tested clinical variables. The Kolmogorov-Smirnov test was used to test for significant differences in predictive power.

### References for the description of the first embodiment

1. Gatta G, Faivre J, Capocaccia R, Ponz de Leon M, EUROCARE working group. Survival of Colorectal Cancer Patients in Europe During the Period 1978-1989. Eur J Cancer 1998; 34:2176-2183.
2. Ragnhammar P, Hafstrom L, Nygren P, Glimelius B, SBU-Group. A systematic overview of chemotherapy effects in colorectal cancer. Acta Oncol 2001; 40:282-308.
3. Simmonds PC. Palliative chemotherapy for advanced colorectal cancer: systematic review and meta-analysis. Colorectal Cancer Collaborative Group. Br Med J 2000; 321:531-535.
4. TNM classification of malignant tumors. 5th ed. Baltimore: Wiley-Liss, 1997.
5. NIH consensus conference. Adjuvant therapy for patients with colon and rectal cancer. JAMA 1990; 264:1444-1450.
6. Köckerling F, Reymond MA, Altendorf-Hofmann A, Dworak O, Hohenberger W. Influence of surgery on metachronous distant metastases and survival in rectal cancer. J Clin Oncol 1998; 16:324-329.
7. Reymond MA, Dworak O, Remke S, Hohenberger W, Kirchner Th, Köckerling F. DCC Protein as a Predictor of Distant Metastases after Curative Surgery in Rectal Cancer. Dis Colon Rectum 1998; 41:755-760.
8. Günther K, Jung A, Völker U, et al. Predictive value of p27-kip1 for the occurrence of distant metastasis in rectal cancer. J Surg Res 2000; 92:78-84.
9. Günther K, Radkow T, Reymond MA, et al. Angiogenese und Dichte dendritischer Zellen korrelieren nicht mit der metachronen Fernmetastasierung bei kurativ operierten Rectumcarcinom. Chirurg 2001; 72:1144-1153.
10. Petersen S, Thamse HD, Nieder C, Petersen C, Baumann M. The results of colorectal cancer treatment by p53 status: treatment-specific overview. Dis Colon Rectum 2001; 44:322-333.
11. Günther K, Dworak O, Remke S, et al. Prediction of Distant Metastases after Curative Surgery for Rectal Cancer. J Surg Res 2002; 103:68-78.
12. Burke HB, Goodman PH, Rosen DB, et al. Artificial neuronal netwoks improve the accuracy of cancer survival prediction. Cancer 1997; 79:857-862.
13. Bottaci L, Drew PJ, Hartley JE, et al. Artificial neural networks applied to outcome prediction for colorectal cancer patients in separate institutions. Lancet 1997; 350:469-472.
14. Snow PB, Kerr DJ, Brandt JM, Rodvold DM. Neural Network and Regression Predictions of 5-Year Survival after Colon Carcinoma Treatment. Cancer 2001; 91:1673-1678.
15. Silverman BW. Density Estimation for Statistics and Data Analysis. Chapman & Hill, 1986:
16. Bishop CM. Neural Networks for Pattern Recognition. Oxford Press, 1995.
17. Ragg T. Bayesan Learning and Evolutionary Parameter Optimization. Adv Artif Intell 2001; 2174.
18. Ragg T. Bayesian learning for sales rates prediction for thousands of retailers. Neurocomputing 2002; (in press).
19. Kaplan EL, Meier P. Non-parametric estimation from incomplete observations. J Am Statistical Assoc 1958; 53:457-481.
20. Mantel N, Haenszel W. Statistical aspects of the analysis of data from restrospective studies of disease. J Natl Cancer Inst 1959; 22:719-748.
21. Empson MB. Statistics in the pathology laboratory : characteristics of diagnostic tests. Pathology 2001; 33:93-95.
22. Press WH. Numerical Recipes in C. Cambridge University Press, 1992.
23. Lakhani SR, Ashworth A. Microarray and histopathological analysis of tumors : the future and the past ? Nature Cancer Rev 2001; 1:151-157.
24. Michel P, Merle V, Chiron A, et al. Postoperetaive management of stage II/III colon cancer : a decision analysis. Gastroenterology 1999; 117:784-793.
25. Riedmiller M. Supervised learning in multilayer perceptrons. Int J Comput Stand Interf 1994; 16.

### References for the description of the second embodiment

Abbruzzese, J. L., Abbruzzese, M. C., Hess, K. R., Raber, M. N., Lenzi, R., and Frost, P. (1994). Unknown primary carcinoma: natural history and prognostic factors in 657 consecutive patients, J Clin Oncol *12*, 1272-80.
Allred, D. C., Mohsin, S. K., and Fuqua, S. A. (2001). Histological and biological evolution of human premalignant breast disease, Endocr Relat Cancer 8, 47-61.
Artandi, S. E., Chang, S., Lee, S. L., Alson, S., Gottlieb, G. J., Chin, L., and DePinho, R. A. (2000). Telomere dysfunction promotes non-reciprocal translocations and epithelial cancers in mice, Nature 406, 641-5.
Aubele, M., Mattis, A., Zitzelsberger, H., Walch, A., Kremer, M., Welzl, G., Hofler, H., and Werner, M. (2000). Extensive ductal carcinoma In situ with small foci of invasive ductal carcinoma: evidence of genetic resemblance by CGH, Int J Cancer *85,* 82-6.
Bishop CM. Neural Networks for Pattern Recognition. Oxford Press, 1995.
Braun, S., Kentenich, C., Janni, W., Hepp, F., de Waal, J., Willgeroth, F., Sommer, H., and Pantel, K. (2000a). Lack of effect of adjuvant chemotherapy on the elimination of single dormant tumor cells in bone marrow of high-risk breast cancer patients, J Clin Oncol *18,* 80-6.
Braun, S., Pantel, K., Muller, P., Janni, W., Hepp, F., Kentenich, C. R., Gastroph, S., Wischnik, A., Dimpfl, T., Kindermann, G., *et al.* (2000b). Cytokeratin-positive cells in the bone marrow and survival of patients with stage I, II, or III breast cancer, N Engl J Med 342, 525-33.
Buerger, H., Otterbach, F., Simon, R., Poremba, C., Diallo, R., Decker, T., Riethdorf, L., Brinkschmidt, C., Dockhorn-Dworniczak, B., and Boecker, W. (1999a). Comparative genomic hybridization of ductal carcinoma in situ of the breast-evidence of multiple genetic pathways, J Pathol *187,* 396-402.
Buerger, H., Otterbach, F., Simon, R., Schafer, K. L., Poremba, C., Diallo, R., Brinkschmidt, C., Dockhorn-Dworniczak, B., and Boecker, W. (1999b). Different genetic pathways in the evolution of invasive breast cancer are associated with distinct morphological subtypes, J Pathol *189,* 521-6.
Clark, E. A., Golub, T. R., Lander, E. S., and Hynes, R. O. (2000). Genomic analysis of metastasis reveals an essential role for RhoC, Nature *406,* 532-5.
Cover TM, Thomas JA. Elements of Information Theory. John Wiley and Sons, 1991.
Demicheli, R. (2001). Tumour dormancy: findings and hypotheses from clinical research on breast cancer, Semin Cancer Biol *11,* 297-306.
DePinho, R. A. (2000). The age of cancer, Nature *408,* 248-54.
Fearon, E. R., and Vogelstein, B. (1990). A genetic model for colorectal tumorigenesis, Cell 61, 759-67.
Empson MB. Statistics in the pathology laboratory: characteristics of diagnostic tests. Pathology 2001;33:93-95.
Fidler, I. J., and Kripke, M. L. (1977). Metastasis results from preexisting variant cells within a malignant tumor, Science 197, 893-5.
Heiss, M. M., Allgayer, H., Gruetzner, K. U., Funke, I., Babic, R., Jauch, K. W., and Schildberg, F. W. (1995). Individual development and uPA-receptor expression of disseminated tumour cells in bone marrow: a reference to early systemic disease in solid cancer, Nat Med 1, 1035-9.
Hiyama, E., Gollahon, L., Kataoka, T., Kuroi, K., Yokoyama, T., Gazdar, A. F., Hiyama, K., Piatyszek, M. A., and Shay, J. W. (1996). Telomerase activity in human breast tumors, J Natl Cancer Inst 88, 116-22.
Hoglund, M., Gisselsson, D., Mandahl, N., Johansson, B., Mertens, F., Mitelman, F., and Sall, T. (2001). Multivariate analyses of genomic imbalances in solid tumors reveal distinct and converging pathways of karyotypic evolution, Genes Chromosomes Cancer *31,* 156-71.
Izbicki, J. R., Hosch, S. B., Pichlmeier, U., Rehders, A., Busch, C., Niendorf, A., Passlick, B., Broelsch, C. E., and Pantel, K. (1997). Prognostic value of immunohistochemically identifiable tumor cells in lymph nodes of patients with completely resected esophageal cancer, N Engl J Med 337, 1188-94.
Janni, W., Hepp, F., Rjosk, D., Kentenich, C., Strobl, B., Schindlbeck, C., Hantschmann, P., Sommer, H., Pantel, K., and Braun, S. (2001). The fate and prognostic value of occult metastatic cells in the bone marrow of patients with breast carcinoma between primary treatment and recurrence, Cancer 92, 46-53.
Jauch, K. W., Heiss, M. M., Gruetzner, U., Funke, I., Pantel, K., Babic, R., Eissner, H. J., Riethmueller, G., and Schildberg, F. W. (1996). Prognostic significance of bone marrow micrometastases in patients with gastric cancer, J Clin Oncol 14, 1810-7.
Klein, C. A. (2000). The biology and analysis of single disseminated tumour cells, Trends Cell Biol *10,* 489-93.
Klein, C. A., Schmidt-Kittler, O. Schardt, J. A., Pantel, K., Speicher, M. R., and Riethmuller, G. (1999). Comparative genomic hybridization, loss of heterozygosity, and DNA sequence analysis of single cells, Proc Natl Acad Sci U S A 96, 4494-9.
Klein, C. A., Seidl, S., Petat-Dutter, K., Offner, S., Geigl, J. B., Schmidt-Kittler, O., Wendler, N., Passlick, B., Huber, R. M., Schlimok, G., *et al.* (2002). Combined transcriptome and genome analysis of single micrometastatic cells, Nat Biotechnol 20, 387-92.
Kuukasjarvi, T., Karhu, R., Tanner, M., Kahkonen, M., Schaffer, A., Nupponen, N., Pennanen, S., Kallioniemi, A., Kallioniemi, O. P., and Isola, J. (1997). Genetic heterogeneity and clonal evolution underlying development of asynchronous metastasis in human breast cancer, Cancer Res 57, 1597-604.
Lindemann, F., Schlimok, G., Dirschedl, P., Witte, J., and Riethmuller, G. (1992). Prognostic significance of micrometastatic tumour cells in bone marrow of colorectal cancer patients, Lancet 340, 685-9.
Mertens, F., Johansson, B., Hoglund, M., and Mitelman, F. (1997). Chromosomal imbalance maps of malignant solid tumors: a cytogenetic survey of 3185 neoplasms, Cancer Res 57, 2765-80.
Nishizaki, T., DeVries, S., Chew, K., Goodson, W. H., 3rd, Ljung, B. M., Thor, A., and Waldman, F. M. (1997). Genetic alterations in primary breast cancers and their metastases: direct comparison using modified comparative genomic hybridization, Genes Chromosomes Cancer 19, 267-72.
Pandis, N., Teixeira, M. R., Adeyinka, A., Rizou, H., Bardi, G., Mertens, F., Andersen, J. A., Bondeson, L., Sfikas, K., Qvist, H., *et al.* (1998). Cytogenetic comparison of primary tumors and lymph node metastases in breast cancer patients, Genes Chromosomes Cancer 22, 122-9.
Pantel, K., Felber, E., and Schlimok, G. (1994). Detection and characterization of residual disease in breast cancer, J Hematother 3, 315-22.
Pantel, K., Izbicki, J., Passlick, B., Angstwurm, M., Haussinger, K., Thetter, O., and Riethmuller, G. (1996). Frequency and prognostic significance of isolated tumour cells in bone marrow of patients with non-small-cell lung cancer without overt metastases, Lancet 347, 649-53.
Pihan, G. A., and Doxsey, S. J. (1999). The mitotic machinery as a source of genetic instability in cancer, Semin Cancer Biol 9, 289-302.
Poremba, C., Bocker, W., Willenbring, H., Schafer, K. L., Otterbach, F., Burger, H., Diallo, R., and Dockhorn-Dworniczak, B. (1998). Telomerase activity in human proliferative breast lesions, Int J Oncol 12, 641-8.
Press WH. Numerical Recipes in C. Cambridge University Press, 1992.
Ragg T. Bayesian learning for sales rates prediction for thousands of retailers. Neurocomputing 2002;43:127-44
Ragg T. Bayesian Learning and Evolutionary Parameter Optimization. In: Baader F, Brewka G, Eiter T (eds.). Advances in Artificial Intelligence, Springer, Heidelberg, 2001;48-62.
Riethmuller, G., and Klein, C. A. (2001). Early cancer cell dissemination and late metastatic relapse: clinical reflections and biological approaches to the dormancy problem in patients, Semin Cancer Biol *11*, 307-11.
Romanov, S. R., Kozakiewicz, B. K., Holst, C. R., Stampfer, M. R., Haupt, L. M., and Tlsty, T. D. (2001). Normal human mammary epithelial cells spontaneously escape senescence and acquire genomic changes, Nature 409, 633-7.
Roylance, R., Gorman, P., Harris, W., Liebmann, R., Barnes, D., Hanby, A., and Sheer, D. (1999). Comparative genomic hybridization of breast tumors stratified by histological grade reveals new insights into the biological progression of breast cancer, Cancer Res 59, 1433-6.
Schlimok, G., Funke, I., Bock, B., Schweiberer, B., Witte, J., and Riethmuller, G. (1990). Epithelial tumor cells in bone marrow of patients with colorectal cancer: immunocytochemical detection, phenotypic characterization, and prognostic significance, J Clin Oncol 8, 831-7.
Schlimok, G., Funke, I., Holzmann, B., Gottlinger, G., Schmidt, G., Hauser, H., Swierkot, S., Warnecke, H. H., Schneider, B., Koprowski, H., and et al. (1987). Micrometastatic cancer cells in bone marrow: in vitro detection with anti-cytokeratin and in vivo labeling with anti-17-1A monoclonal antibodies, Proc Natl Acad Sci U S A 84, 8672-6.
Thorban, S., Rosenberg, R., Busch, R., and Roder, R. J. (2000). Epithelial cells in bone marrow of oesophageal cancer patients: a significant prognostic factor in multivariate analysis, Br J Cancer *83,* 35-9.
Tirkkonen, M., Tanner, M., Karhu, R., Kallioniemi, A., Isola, J., and Kallioniemi, O. P. (1998). Molecular cytogenetics of primary breast cancer by CGH, Genes Chromosomes Cancer 21, 177-84.
Tsao, J., Zhao, Y., Lukas, J., Yang, X., Shah, A., Press, M., and Shibata, D. (1997). Telomerase activity in normal and neoplastic breast, Clin Cancer Res 3, 627-31.
Uhr, J. W., Scheuermann, R. H., Street, N. E., and Vitetta, E. S. (1997). Cancer dormancy: opportunities for new therapeutic approaches, Nat Med 3, 505-9.
Vogelstein, B., Fearon, E. R., Hamilton, S. R., Kern, S. E., Preisinger, A. C., Leppert, M., Nakamura, Y., White, R., Smits, A. M., and Bos, J. L. (1988). Genetic alterations during colorectal-tumor development, N Engl J Med 319, 525-32.
Vogelstein, B., and Kinzler, K. W. (1993). The multistep nature of cancer, Trends Genet 9, 138-41.
Waldman, F. M., DeVries, S., Chew, K. L., Moore, D. H., 2nd, Kerlikowske, K., and Ljung, B. M. (2000). Chromosomal alterations in ductal carcinomas in situ and their in situ recurrences, J Natl Cancer Inst 92, 313-20.
Weckermann, D., Muller, P., Wawroschek, F., Harzmann, R., Riethmuller, G., and Schlimok, G. (2001). Disseminated cytokeratin positive tumor cells in the bone marrow of patients with prostate cancer: detection and prognostic value, J Urol 166, 699-703.
Weinberg, R. A. (1989). Oncogenes, antioncogenes, and the molecular bases of multistep carcinogenesis, Cancer Res 49, 3713-21.
Willis, R. A. (1952). The spread of tumours in the human body, Vol IX, 447, 28 S.: III (London, Butterworth).

### Supp. Table I Complete list of variables

- 1: Registration number
- 2: Date of birth
- 3: Year of entry into study
- 4: Sex
- 5: Localisation of tumor
- 6: WHO histological classification
- 7: Distant metastasis
- 8: Synchronous existence of different malignancies
- 9: Different malignancies before study
- 10: Known polyps in colon
- 11: First date of diagnosis of polyps (in years before study entry)
- 12: Precancerosis/ risk (FAP, Polyposis coli, etc.)
- 13: ECOG performance status
- 14: Risk group according to ASA at admission
- 15: Major clinical symptoms
- 16: Major pre-operative complications
- 17: CEA level before surgery
- 18: Distance to linea anocutanea (mm)
- 19: Endorectal sonography of the primary tumor
- 20: Endorectal sonographic examination of the regional lymph nodes
- 21: Mason stage (rectal carcinoma)
- 22: Number of malignancies
- 23: Primary localisation of tumor
- 24: Localisation of tumor within rectum
- 25: Intraoperative diagnostic findings
- 26: Tumor stenosis
- 27: Inner syrinx
- 28: Peritumorous abscess
- 29: Perforation
- 30: Transsection or rupture of tumor
- 31: Emergency surgery
- 32: Limited surgery prior to tumor operation
- 33: Operation method
- 34: Restoration of continuity of colon
- 35: Protective anus praeter
- 36: En-bloc resection
- 37: Extension of operation due to tumor spreading
- 38: Which organ was resected additionally_1
- 39: Which organ was resected additionally_2
- 40: Which organ was resected additionally_3
- 41: Extension of operation due to other reasons
- 42: Which organ was resected additionally_A
- 43: Which organ was resected additionally_B
- 44: Which organ was resected additionally_C
- 45: Insufficiency of anastomosis
- 46: Therapy of insufficiency of anastomosis

- 47: Further postoperative complications_1
- 48: Further postoperative complications_2
- 49: Further postoperative complications_3
- 50: Therapy of worst complications
- 51: Grading
- 52: Degree of malignancy
- 53: Invasion of lymphatic vessels
- 54: Invasion of venous vessels
- 55: Invasion of perineural tissue
- 56: Extension of inflammatory stroma reaction
- 57: Type of inflammatory stroma reaction
- 58: Lymphocyte aggregates around tumor site
- 59: Stromal fibrosis
- 60: Histological tumor margin free of tumor cells
- 61: Peritoneal cytolgy
- 62: Distance to linea ano-cutanea established by pathologist (mm)
- 63: Macroscopic tumor type
- 64: Macroscopic infestation of serosa
- 65: Tumor form
- 66: Tumor size (mm)
- 67: Tumor size; second diameter (mm)
- 68: Macroscopic tumor size (mm)
- 69: Intestinal circumference at the site of maximal tumor size
- 70: Distance to aboral resection margin (mm)
- 71: Distance to oral resection margin (mm)
- 72: Measurement method
- 73: Minimal distance from surface (mm)
- 74: Infiltration depth
- 75: pT-Classification
- 76: Oral resp. first resection marginal (histologically)
- 77: Oral resp. second resection margin (histologically)
- 78: Surface resection margin (histologically)
- 79: Number of histologically examined lymph nodes
- 80: Number of neoplastic lymph nodes
- 81: Arteria rectalis superior
- 82: Arteria sigmoidea
- 83: Arteria mesenterica inferior
- 84: Lateral rectal drain region
- 85: Arteria colica sinistra
- 86: Arteria colica media
- 87: Arteria colica dextra
- 88: Arteria ileocolica
- 89: pN-Classification
- 90: R-Classification
- 91: AJCC/UICC stage
- 92: Genesis of tumor
- 93: Tubular adenoma
- 94: Tubular adenoma with high level dysplasia
- 95: Tubulo-villous adenoma
- 96: Tubulo-villous adenoma with high level dysplasia
- 97: Villous adenoma
- 98: Villous adenoma with high level dysplasia
- 99: Hyperplastic polyp
- 100: Carcinoid
- 101: Dysplasia during colitis ulcerosa
- 102: Other changes
- 103: Localisation of distant metastasis
- 104: Surgical therapy
- 105: Type of operation
- 106: Different therapy of metastasis
- 107: First treated tumor at finish
- 108: Date of the last follow-up examination
- 109: Presence of local recurrence in months after study entry
- 110: Local recurrence locally radically operated
- 111: Presence of distant metastasis when local recurrence diagnosed
- 112: Dukes stage of local recurrence
- 113: First treated tumor at finish
- 114: Date of last follow-up examination
- 115: Survival time in months
- 116: Dead or alive
- 117: Carcinoma at primary tumor site
- 118: Adenoma at different licalisation
- 119: Adenoma with high dysplasia at different site
- 120: Colorectal carcinoma at different site
- 121: Different carcinoma during study
- 122: First treated tumor at finish
- 123: Cause of death
- 124: Carcinoma at primary tumor site

## Claims

1. Method for predicting the development of a disease and/or identifying high-risk patients, the method having the following steps:
(a) providing molecular genetic data and/or clinical data,
(b) pre-processing the data,
(c) selecting a predetermined number of variables out of the provided data according to their combined/mutual information content,
(d) automatically generating prediction data by means of machine learning.

2. Method according to claim 1 for predicting the development of tumors.

3. Method according to claim 1 or 2, wherein the generated prediction data comprises the development of distant metastases and/or the overall survival rate after a predetermined number of years.

4. Method according to any one of the preceding claims, wherein the provided clinical data comprise variables such as UICC stage, type of surgical procedure, age, tumor grading, depth of tumor infiltration, occurrence of post-operative complications and/or the presence of venous invasion.

5. Method according to any one of the preceding claims, wherein the provided molecular genetic data comprise variables defining the genomic organization of cancer cells.

6. Method according to any one of the preceding claims, wherein the provided molecular genetic data comprise variables defining the genomic organization of single disseminated cancer cells.

7. Method according to any one of the preceding claims, wherein pre-processing the data comprises transformation of the provided data into class-conditional probabilities.

8. Method according to any one of the preceding claims, wherein at most 20 variables, preferably at most 10 variables are selected.

9. Method according to any one of the preceding claims, wherein the machine learning system is an artificial neural network.

10. Method according to claim 9, wherein the artificial neural network is trained using the Bayesian evidence framework applying a cross-entropy error function combined with a weight decay regularizer.

11. Computer program comprising program code means for performing the method of any one of the preceding claims when the program is run on a computer.

12. Computer program product comprising program code means stored on a computer readable medium for performing the method of any one of claims 1-10 when said program product is run on a computer.

13. Computer system, particularly for performing the method of any one of the claims 1-10, comprising:
(a) means for providing molecular genetic data and/or clinical data,
(b) means for pre-processing the data,
(c) means for selecting a predetermined number of variables out of the provided data according to their combined/mutual information content,
(d) means for automatically generating prediction data by means of machine learning.

14. Computer system according to claim 13, wherein the system comprises means for carrying out the method steps as recited in one or more of claims 1 to 9.

15. Use of the method according to any one of claims 1-10 or a system according to claim 13 or 14 for deciding on an adjuvant therapy for an individual patient.
